# EUROPEAN PATENT SPECIFICATION

(11) **EP 1 567 539 B1**
(45) Date of publication and mention of the grant of the patent: **23.09.2009**
(21) Application number: 03783121.1
(22) Date of filing: 04.11.2003
(51) Int. Cl.: C12N 15/11, A61K 48/00, A61K 31/713

(54) **ALLELE-SPECIFIC RNA INTERFERENCE**
ALLELSPEZIFISCHE RNA-INTERFERENZ
INTERFERENCE D'ARN PROPRE A UN ALLELE

(30) Priority: 04.11.2002 US 423507 P; 18.07.2003 US 488283 P
(43) Date of publication of application: 31.08.2005
(73) Proprietor: University of Massachusetts, Boston, MA 02108 (US)
(72) Inventor: XU, Zuoshang, North Grafton, MA 01536 (US); ZAMORE, Phillip, D., Northboro, MA 01532 (US)
(74) Representative: Kirkham, Nicholas Andrew
(86) International application number: PCT/US2003/035009
(87) International publication number: WO 2004/042027

(56) References cited:
- WO-A-98/48009
- WO-A-03/080807
- US-A1- 2003 180 756
- BRUMMELKAMP T R ET AL: "STABLE SUPPRESSION OF TUMORIGENICITY BY VIRUS-MEDIATED RNA INTERFERENCE" CANCER CELL, XX, US, vol. 2, no. 3, September 2002 (2002-09), pages 243-247, XP009006464 ISSN: 1535-6108
- FLUITER KEES ET AL: "Tumor genotype-specific growth inhibition in vivo by antisense oligonucleotides against a polymorphic site of the large subunit of human RNA polymerase II." CANCER RESEARCH. 1 APR 2002, vol. 62, no. 7, 1 April 2002 (2002-04-01), pages 2024-2028, XP002380704 ISSN: 0008-5472
- SUI G ET AL: "A DNA vector-based RNAi technology to suppress gene expression in mammalian cells" PROCEEDINGS OF THE NATIONAL ACADEMY OF SCIENCES OF USA, NATIONAL ACADEMY OF SCIENCE, WASHINGTON, DC, US, vol. 99, no. 8, 16 April 2002 (2002-04-16), pages 5515-5520, XP002311179 ISSN: 0027-8424
- CAPLEN NATASHA J ET AL: "Rescue of polyglutamine-mediated cytotoxicity by double-stranded RNA-mediated RNA interference." HUMAN MOLECULAR GENETICS. 15 JAN 2002, vol. 11, no. 2, 15 January 2002 (2002-01-15), pages 175-184, XP002380705 ISSN: 0964-6906
- VICTOR MARTIN ET AL: "HAT activity is essential for CBP-1-dependent transcription and differentiation in Caenorhabditis elegans." EMBO REPORTS. JAN 2002, vol. 3, no. 1, January 2002 (2002-01), pages 50-55, XP002380706 ISSN: 1469-221X
- MILLER V.M. ET AL.: 'Allele-specific silencing of dominant disease genes' PNAS vol. 100, no. 12, 10 June 2003, pages 7195 - 7200, XP002977550

## Description

### BACKGROUND

Diseases caused by dominant, gain-of-function gene mutations develop in heterozygotes bearing one mutant and one wild type copy of the gene. Some of the best-known diseases of this class are common neurodegenerative diseases, including Alzheimer's disease, Huntington's disease, Parkinson's disease and amyotrophic lateral sclerosis (ALS; "Lou Gehrig's disease") (Taylor et al., 2002). In these diseases, the exact pathways whereby the mutant proteins cause cell degeneration are not clear, but the origin of the cellular toxicity is known to be the mutant protein.

Mutations in SOD1 cause motor neuron degeneration that leads to ALS, because the mutant protein has acquired some toxic property (Cleveland et al., 2001). Neither the nature of this toxic property nor the downstream pathway that leads to the eventual motor neuron degeneration is understood. In mice, only expression of the mutant SOD1, but not elimination of SOD1 by gene knockout, causes ALS. Nonetheless, the gene knockout mice develop numerous abnormalities including reduced fertility (Matzuk et al., 1990), motor axonopathy (Shefner et al., 1999), age-associated loss of cochlear hair cells (McFadden et al., 2001) and neuromuscular junction synapses (Flood et al., 1999), and enhanced susceptibility to a variety of noxious assaults, such as excitotoxicity, ischemia, neurotoxins and irradiation, on the CNS and other systems (Matz et al., 2000; Kondo et al., 1997; Kawase et al., 1999; Behndig et al., 2001). Given the toxicity of the mutant and the functional importance of the wild-type protein, the ideal therapy for this disease would selectively block the expression of the mutant protein while retaining expression of the wild type.

Cancer Cell: September 2002, Vol 2 243-247, Brummelkamp *et al*, discloses stable suppression of tumorigenicity by virus-mediated RNA interference.

Cancer Research 2024-028 April 2002, Fluiter *et al*, discloses tumor genotype-specific inhibition *in vivo* by antisense oligonucleotides against a polymorphic site of the large subunit of human RNA polymerase II.

WO-A-98/48009 discloses materials and methods for ribozyme treatment of retinal diseases.

### SUMMARY

The present invention relates to novel methods for treating dominant gain-of-function disease. In particular, the invention provides methods for the selective destruction of mutant mRNA's transcribed from gain-of-function genes, thus preventing the production of mutant proteins encoded by such genes. The invention is based in part on the discovery that both small interfering RNAs (siRNAs) and small hairpin RNAs (shRNAs) can be designed to selectively inhibit expression of a mutant allele, *e*.*g*., G85R SOD1 or G93A SOD1, while preserving expression of the wild-type protein, with single-nucleotide specificity.

The methods of the invention utilize RNA interference technology (RNAi) against selected point mutations occurring in a single allele in a mutant gene *e*.*g.*, the point mutation in the copper zinc superoxide dismutase (SOD1) gene associated with amyotrophic lateral sclerosis (ALS). RNAi can mediate sequence-selective suppression of gene expression in a wide variety of eukaryotes by introducing short RNA duplexes (called small interfering RNAs or siRNAs) with sequence homologies to the target gene (Caplen et al., 2001; Elbashir et al., 2001c). siRNA duplexes or vectors expressing shRNAs of the present invention can be used to silence the expression of a toxic mutant gene selectively *e*.*g*., the SOD1 mutant protein, thereby allowing the wild-type SOD1 allele to continue functioning.

The invention is also based on the discovery of new artificial, engineered RNA precursors, that when expressed in a cell, *e*.*g*., *in-vivo*, are processed by the cell to produce targeted siRNAs that selectively silence mutant alleles of target genes (by targeting specific mRNAs for cleavage) using the cell's own RNAi pathway. By introducing nucleic acid molecules that encode these engineered RNA precursors into cells *in-vivo* with appropriate regulatory sequences (*e*.*g.*, a transgene in a vector such as a plasmid), expression of the engineered RNA precursors can be selectively controlled both temporally and spatially, *i*.*e*., at particular times and/or in particular tissues, organs, or cells.

In one aspect, the invention features a method of inhibiting expression of a target allele in a cell comprising at least two different alleles of a gene by administering to the cell an siRNA specific for the target allele. In one embodiment, the target allele is correlated with a disorder associated with a dominant, gain of function mutation. In another embodiment, the disorder is amyotrophic lateral sclerosis, Huntington's disease, Alzheimer's disease, or Parkinson's disease.

In another aspect, the invention features a method of treating a subject having a disorder correlated with the presence of a dominant, gain-of-function mutant allele, the method comprising administering to the subject a therapeutically effective amount of an siRNA specific for the mutant allele. In one embodiment, the siRNA is targeted to the gain-of-function mutation. In another embodiment, the disorder is amyotrophic lateral sclerosis, Huntington's disease, Alzheimer's disease, or Parkinson's disease.

In one embodiment, the disease is amyotrophic lateral sclerosis. In a further embodiment, the allele is a SOD1 mutant allele.

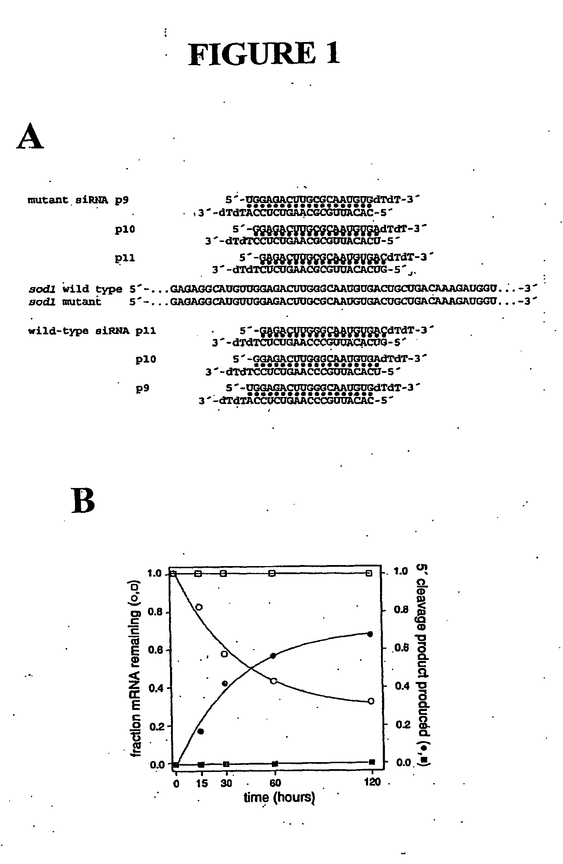

In one embodiment, the siRNA targets a mutant SOD1 allele (SEQ ID NO:8) and comprises or consists of a mutant siRNA sequence as set forth in Figure 1A with P10 (SEQ ID NO:4) being preferred, followed by P9 (SEQ ID NO:2), followed by P11 (SEQ ID NO:6).

In another embodiment, the siRNA (*e*.*g*., a control siRNA) targets a wild-type SOD 1 allele and comprises or consists of a wild-type siRNA sequence as set forth in Figure 1A with P9 (SEQ ID NO:14) or P10 (SEQ ID NO:12) being preferred, followed by P11 (SEQ ID NO:10).

In another aspect, the invention provides an siRNA comprising a sequence as set forth in Figure 1A.

In another aspect, the invention provides a p10 mutant siRNA comprising the sequence as set forth in Figure 1A (SEQ ID NO: 4).

In another aspect, the invention provides a p9 mutant siRNA comprising the sequence as set forth in Figure 1A (SEQ ID NO: 2).

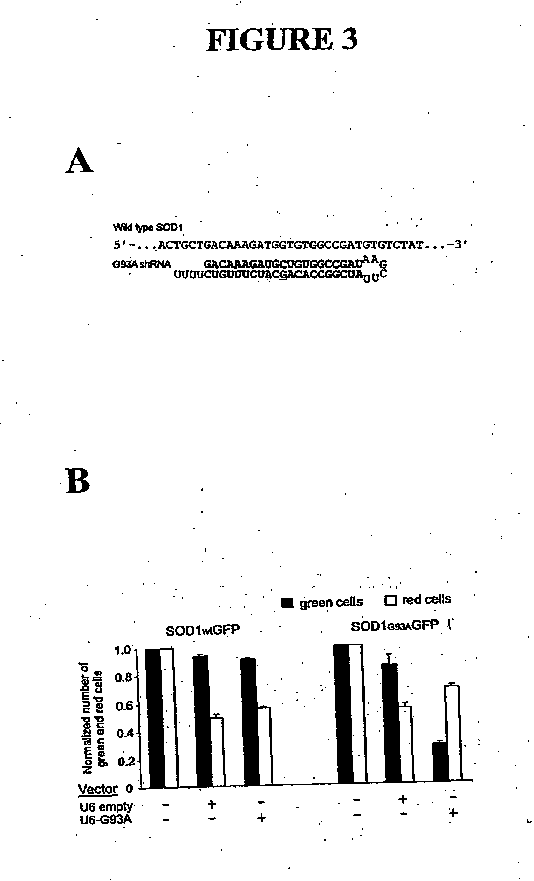

In another aspect, the invention provides a G93A SOD1 shRNA comprising the sequence as set forth in Figure 3A (SEQ ID NO: 16), as well as expression constructs comprising the shRNAs of the invention.

In another aspect, the invention provides therapeutic compositions comprising the siRNAs and/or shRNAs of the invention, and a pharmaceutically acceptable carrier.

Other features and advantages of the invention will be apparent from the following detailed description, and from the claims.

### DESCRIPTION OF DRAWINGS

Figure 1. siRNA duplexes can discriminate between mutant and wild-type SOD1 *in-vitro*. (A) siRNA duplexes used: mutant siRNA P11 (SEQ ID NO: 5, sense; SEQ ID NO: 6, anti-sense or guide), mutant siRNAP10 (SEQ ID NO: 3, sense; SEQ ID NO: 4, anti-sense or guide), mutant siRNA P9 (SEQ ID NO: 1, sense; SEQ ID NO: 2 anti-sense or guide), SOD1 wild-type target (SEQ ID NO: 7), SOD1 mutant target (SEQ ID NO: 8), wild-type siRNAP11 (SEQ ID NO: 9 sense; SEQ ID NO: 10, anti-sense or guide), wild-type siRNAP10 (SEQ ID NO: 11, sense; SEQ ID NO: 12, anti-sense or guide), wild-type siRNAP9 (SEQ ID NO: 13, sense; SEQ ID NO: 14, anti-sense or guide) (B) Mutant siRNAp10 targets mutant but not wild-type SOD1 mRNA for destruction by the RNAi pathway.
Figure 2. Selective inhibition of mutant SOD1 G85R expression by siRNA in Hela cells. SOD1wtGFP or G85R-GFP were cotransfected with various siRNAs. DsRed was cotransfected as a transfection control. Green and red fluorescent cells were quantified using FACS. (A) Relative number of green (solid bars) and red (open bars) cells in the transfections (n=3). Error bars represent standard deviation.
Figure 3. Selective inhibition of mutant SOD1 G93A expression by U6-G93A vector in Hela cells. (A) Design of the G93A shRNA (SEQ ID NO: 16), (B) SOD1wtGFP or SOD1 G93A-GFP were cotransfected with U6-empty or U6-G93A. DsRed was cotransfected as a transfection control. Green and red fluorescent cells were quantified using FACS. Results from four experiments were averaged. Error bars represent standard deviation.
Figure 4. Selective inhibition of mutant SOD 1 expression by siRNA and U6-G93A vector in neuroblastoma N2a cells. (A) siRNA against G85R (n=4), (B) U6-G93A vector (n=3). Error bars represent standard deviation.
Figure 5. Selective inhibition of mutant SOD1 G85R but not the wild type SOD1 expression by siRNA in the same cells. (A) Relative levels of SOD 1 measured from protein blots of transfected Hela cells detecting mutant SOD1 G85R-GFP (average of 4 transfections). Error bars are standard error.
Figure 6. Selective inhibition of mutant SOD1 expression by U6-G93A vector in vivo. (A) SOD1 G93A-GFP were co-transfected with a C-terminal myc tagged wild-type human SOD1 in mice using the hydrodynamic transfection method. The relative band intensities on SDA-PAGE were quantified. The ratio of SOD1 G93A-GFP to wild type SODlmyc are shown. Eight animals were used in each group. The U6-G93A group is significantly different from the other two groups (p < 0.05).
Figure 7 is the Genbank entry for human SOD-1 protein, Accession No. NP_000445, showing the deduced amino acid sequence of wild-type SOD-1 (SEQ ID NO:18).

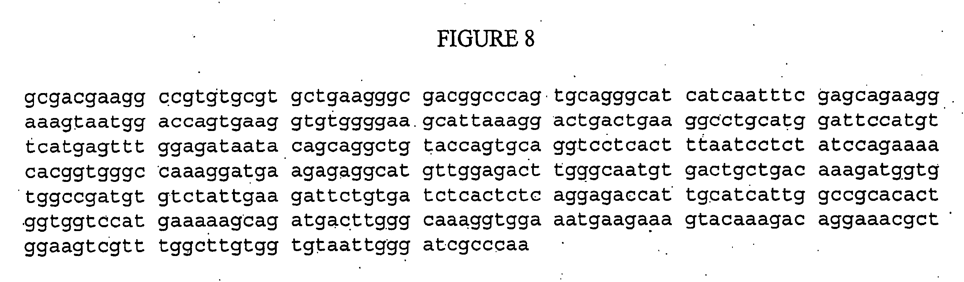

Figure 8 is the Genbank entry for human SOD-1 mRNA, Accession No. NM_000454, showing the nucleotide sequence of wild-type SOD-1 (SEQ ID NO:17).

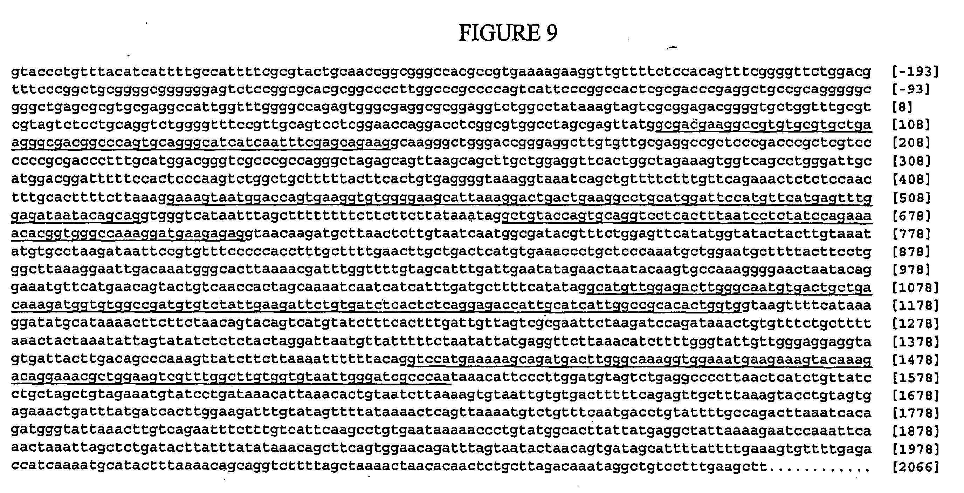

Figure 9 is the SOD1 genomic sequence (SEQ ID NO: 19)

### DETAILED DESCRIPTION

Mutations in copper zinc superoxide dismutase (SOD1) gene cause a subset of amyotrophic lateral sclerosis, a neurodegenerative disease that leads to motor neuron degeneration, paralysis and death (Brown and Robberecht, 2001; Siddique and Lalani, 2002). It has been well established that mutant SOD1 causes motor neuron degeneration by acquisition of a toxic property (Cleveland and Rothstein, 2001). However, neither the molecular basis of this toxic property nor mechanism that leads to motor neuron death is understood. Because of this incomplete understanding of the disease mechanism, rational design of therapy has not produced robust efficacious outcomes. On the other hand, because the toxicity that kills motor neurons originates from the mutated protein (Cleveland and Rothstein, 2001), decrease of the mutant protein should alleviate or even prevent the disease. RNA interference (RNAi) technology can be used to achieve this goal.

The present invention is based on the discovery that siRNA and shRNA can selectively inhibit the expression of a mutant allele, even when the mutant mRNA differs from wild-type by only a single nucleotide, as is the case with certain mutations, e.g., mutations of SOD1 correlated with ALS. These methods are applicable to the treatment of diseases that are caused by dominant, gain-of-function type of gene mutations, including, but not limited to, ALS. The siRNAs of the present invention are capable of single nucleotide discrimination and selectively down-regulating expression of their target genes.

The methods of the invention utilize RNA interference technology (RNAi) against selected point mutations occurring in a single allele in the mutant gene *e*.*g*., the point mutation in the copper zinc superoxide dismutase (SOD1) gene associated with amyotrophic lateral sclerosis (ALS). RNAi can mediate sequence-selective suppression of gene expression in a wide variety of eukaryotes by introducing short RNA duplexes (called small interfering RNAs or siRNAs) with sequence homologies to the target gene (Caplen et al., 2001; Elbashir et al., 2001c). siRNA duplexes or vectors expressing shRNAs of the present invention can be used to silence the expression of a toxic mutant gene selectively *e*.*g*., the SOD1 mutant protein, thereby allowing the wild-type SOD1 allele to continue functioning.

Sequence-selective, post-transcriptional inactivation of gene expression can be achieved in a wide variety of eukaryotes by introducing double-stranded RNA corresponding to the target gene, a phenomenon termed RNAi (Hutvagner and Zamore, 2002; Hannon, G. J., 2002; McManus and Sharp, 2002). RNAi methodology has been extended to cultured mammalian cells (Caplen et al, 2001; Elbashir et al., 2001). This approach takes advantage of the discovery that siRNA, an intermediate in the RNAi pathway, can trigger the degradation of mRNA corresponding to the siRNA sequence. Furthermore, shRNA transcribed *in-vivo* can trigger degradation of target RNAs complementary to the sequence of the shRNA stem, because shRNA is processed into siRNA in cells (Paul et al., 2002; Lee et al., 2002; Paddison et al., 2002; Sui et al., 2002; Yu et al., 2002; McManus et al., 2002; Zeng et al., 2002; Brummelkamp et al., 2002; Miyagishi et al., 2002; Jacque et al., 2002). The present applicants demonstrate that siRNA duplexes or viruses expressing shRNA can be used to preferentially block the expression of a mutant allele, while preserving the expression of a co-expressed wild type allele.

The vast majority of ALS-associated SOD1 mutations are single nucleotide point mutations resulting in single amino acid changes (ALS online database for ALS genetic (*SOD1, ALS and other*) mutations). Thus, to selectively silence the expression of the mutant, but not the wild type, single nucleotide specificity is required. Applicants have now shown that single nucleotide discrimination is achievable in mammalian cells.

So that the invention maybe more readily understood, certain terms are first defined:

An "isolated nucleic acid molecule or sequence" is a nucleic acid molecule or sequence that is not immediately contiguous with both of the coding sequences with which it is immediately contiguous (one on the 5' end and one on the 3' end) in the naturally occurring genome of the organism from which it is derived. The term therefore includes, for example, a recombinant DNA or RNA that is incorporated into a vector; into an autonomously replicating plasmid or virus; or into the genomic DNA of a prokaryote or eukaryote, or which exists as a separate molecule (*e*.*g*., a cDNA or a genomic DNA fragment produced by PCR or restriction endonuclease treatment) independent of other sequences. It also includes a recombinant DNA that is part of a hybrid gene encoding an additional polypeptide sequence.

The term "nucleoside" refers to a molecule having a purine or pyrimidine base covalently linked to a ribose or deoxyribose sugar. Exemplary nucleosides include adenosine, guanosine, cytidine, uridine and thymidine. Additional exemplary nucleosides include inosine, 1-methyl inosine, pseudouridine, 5,6-dihydrouridine, ribothymidine, ²N-methylguanosine and ^{2,2}N,N-dimethylguanosine (also referred to as "rare" nucleosides). The term "nucleotide" refers to a nucleoside having one or more phosphate groups joined in ester linkages to the sugar moiety. Exemplary nucleotides include nucleoside monophosphates, diphosphates and triphosphates. The terms "polynucleotide" and "nucleic acid molecule" are used interchangeably herein and refer to a polymer of nucleotides joined together by a phosphodiester linkage between 5' and 3' carbon atoms.

The term "RNA" or "RNA molecule" or "ribonucleic acid molecule" refers to a polymer of ribonucleotides. The term "DNA" or "DNA molecule" or deoxyribonucleic acid molecule" refers to a polymer of deoxyribonucleotides. DNA and RNA can be synthesized naturally (*e*.*g*., by DNA replication or transcription of DNA, respectively). RNA can be post-transcriptionally modified. DNA and RNA can also be chemically synthesized. DNA and RNA can be single-stranded (*i*.*e*., ssRNA and ssDNA, respectively) or multi-stranded (*e*.*g*., double stranded, *i*.*e*., dsRNA and dsDNA, respectively). "mRNA" or "messenger RNA" is single-stranded RNA that specifies the amino acid sequence of one or more polypeptide chains. This information is translated during protein synthesis when ribosomes bind to the mRNA.

The term "engineered," as in an engineered RNA precursor, or an engineered nucleic acid molecule, indicates that the precursor or molecule is not found in nature, in that all or a portion of the nucleic acid sequence of the precursor or molecule is created or selected by man. Once created or selected, the sequence can be replicated, translated, transcribed, or otherwise processed by mechanisms within a cell. Thus, an RNA precursor produced within a cell from a transgene that includes an engineered nucleic acid molecule is an engineered RNA precursor.

As used herein, the term "small interfering RNA" ("siRNA") (also referred to in the art as "short interfering RNAs") refers to an RNA (or RNA analog) comprising between about 10-50 nucleotides (or nucleotide analogs) which is capable of directing or mediating RNA interference. Preferably, a siRNA comprises between about 15-30 nucleotides or nucleotide analogs, more preferably between about 16-25 nucleotides (or nucleotide analogs), even more preferably between about 18-23 nucleotides (or nucleotide analogs), and even more preferably between about 19-22 nucleotides (or nucleotide analogs) (*e*.*g*., 19, 20, 21 or 22 nucleotides or nucleotide analogs). The term "short" siRNA refers to a siRNA comprising ∼21 nucleotides (or nucleotide analogs), for example, 19, 20, 21 or 22 nucleotides. The term "long" siRNA refers to a siRNA comprising ∼24-25 nucleotides, for example, 23, 24, 25 or 26 nucleotides. Short siRNAs may, in some instances, include fewer than 19 nucleotides, e.g., 16, 17 or 18 nucleotides, provided that the shorter siRNA retains the ability to mediate RNAi. Likewise, long siRNAs may, in some instances, include more than 26 nucleotides, provided that the longer siRNA retains the ability to mediate RNAi absent further processing, *e*.*g*., enzymatic processing, to a short siRNA.

The term "nucleotide analog" or "altered nucleotide" or "modified nucleotide" refers to a non-standard nucleotide, including non-naturally occurring ribonucleotides or deoxyribonucleotides. Preferred nucleotide analogs are modified at any position so as to alter certain chemical properties of the nucleotide yet retain the ability of the nucleotide analog to perform its intended function. Examples of positions of the nucleotide which may be derivitized include the 5 position, *e*.*g*., 5-(2-amino)propyl uridine, 5-bromo uridine, 5-propyne uridine, 5-propenyl uridine, etc.; the 6 position, *e*.*g*., 6-(2-amino)propyl uridine; the 8-position for adenosine and/or guanosines, *e*.*g*., 8-bromo guanosine, 8-chloro guanosine, 8-fluoroguanosine, etc. Nucleotide analogs also include deaza nucleotides, *e*.*g*., 7-deaza-adenosine; O- and N-modified (*e*.*g*., alkylated, *e*.*g*., N6-methyl adenosine, or as otherwise known in the art) nucleotides; and other heterocyclically modified nucleotide analogs such as those described in Herdewijn, Antisense Nucleic Acid Drug Dev., 2000 Aug. 10(4):297-310.

Nucleotide analogs may also comprise modifications to the sugar portion of the nucleotides. For example the 2' OH-group may be replaced by a group selected from H, OR, R, F, Cl, Br, I, SH, SR, NH₂, NHR, NR₂, COOR, or OR, wherein R is substituted or unsubstituted C₁ -C₆ alkyl, alkenyl, alkynyl, aryl, etc. Other possible modifications include those described in U.S. Patent Nos. 5,858,988, and 6,291,438.

The phosphate group of the nucleotide may also be modified, *e*.*g*., by substituting one or more of the oxygens of the phosphate group with sulfur (e.g., phosphorothioates), or by making other substitutions which allow the nucleotide to perform its intended function such as described in, for example, Eckstein, Antisense Nucleic Acid Drug Dev. 2000 Apr. 10(2):117-21, Rusckowski et al. Antisense Nucleic Acid Drug Dev. 2000 Oct. 10(5):333-45, Stein, Antisense Nucleic Acid Drug Dev. 2001 Oct. 11(5): 317-25, Vorobjev et al. Antisense Nucleic Acid Drug Dev. 2001 Apr. 11(2):77-85, and U.S. Patent No. 5,684,143. Certain of the above-referenced modifications (e.g., phosphate group modifications) preferably decrease the rate of hydrolysis of, for example, polynucleotides comprising said analogs *in-vivo* or *in-vitro*.

As used herein, the term "antisense strand" of an siRNA or RNAi agent *e*.*g*., an antisense strand of an siRNA duplex or siRNA sequence, refers to a strand that is substantially complementary to a section of about 10-50 nucleotides, *e*.*g*., about 15-30, 16-25, 18-23 or 19-22 nucleotides of the mRNA of the gene targeted for silencing. The antisense strand or first strand has sequence sufficiently complementary to the desired target mRNA sequence to direct target-specific RNA interference (RNAi), *e*.*g*., complementarity sufficient to trigger the destruction of the desired target mRNA by the RNAi machinery or process. The term "sense strand" or "second strand" of an siRNA or RNAi agent *e*.*g*., an antisense strand of an siRNA duplex or siRNA sequence, refers to a strand that is complementary to the antisense strand or first strand. Antisense and ssense strands can also be referred to as first or second strands, the first or second strand having complementarity to the target sequence and the respective second or first strand having complementarity to said first or second strand.

As used herein, the term "guide strand" refers to a strand of an RNAi agent, e.g., an antisense strand of an siRNA duplex or siRNA sequence, that enters into the RISC complex and directs cleavage of the target mRNA.

As used herein, the "5' end", as in the 5' end of an antisense strand, refers to the 5' terminal nucleotides, e.g., between one and about 5 nucleotides at the 5' terminus of the antisense strand. As used herein, the "3' end", as in the 3' end of a sense strand, refers to the region, *e*.*g*., a region of between one and about 5 nucleotides, that is complementary to the nucleotides of the 5' end of the complementary antisense strand

The term "oligonucleotide" refers to a short polymer of nucleotides and/or nucleotide analogs. The term "RNA analog" refers to an polynucleotide (*e*.*g*., a chemically synthesized polynucleotide) having at least one altered or modified nucleotide as compared to a corresponding unaltered or unmodified RNA but retaining the same or similar nature or function as the corresponding unaltered or unmodified RNA. As discussed above, the oligonucleotides may be linked with linkages which result in a lower rate of hydrolysis of the RNA analog as compared to an RNA molecule with phosphodiester linkages. For example, the nucleotides of the analog may comprise methylenediol, ethylene diol, oxymethylthio, oxyethylthio, oxycarbonyloxy, phosphorodiamidate, phophoroamidate, and/or phosphorothioate linkages. Preferred RNA analogues include sugar- and/or backbone-modified ribonucleotides and/or deoxyribonucleotides. Such alterations or modifications can further include addition of non-nucleotide material, such as to the end(s) of the RNA or internally (at one or more nucleotides of the RNA). An RNA analog need only be sufficiently similar to natural RNA that it has the ability to mediate (mediates) RNA interference.

As used herein, the term "RNA interference" ("RNAi") refers to a selective intracellular degradation of RNA. RNAi occurs in cells naturally to remove foreign RNAs (*e*.*g*., viral RNAs). Natural RNAi proceeds via fragments cleaved from free dsRNA which direct the degradative mechanism to other similar RNA sequences. Alternatively, RNAi can be initiated by the hand of man, for example, to silence the expression of target genes.

An RNAi agent having a strand which is "sequence sufficiently complementary to a target mRNA sequence to direct target-specific RNA interference (RNAi)" means that the strand has a sequence sufficient to trigger the destruction of the target mRNA by the RNAi machinery or process.

As used herein, the term "isolated RNA" (e.g.," isolated siRNA" or "isolated siRNA precursor") refers to RNA molecules which are substantially free of other cellular material, or culture medium when produced by recombinant techniques, or substantially free of chemical precursors or other chemicals when chemically synthesized.

A "target gene" is a gene whose expression is to be selectively inhibited or "silenced." This silencing is achieved by cleaving the mRNA of the target gene by an siRNA that is created from an engineered RNA precursor by a cell's RNAi system. One portion or segment of a duplex stem of the RNA precursor is an anti-sense strand that is complementary, *e*.*g*., fully complementary, to a section of about 18 to about 40 or more nucleotides of the mRNA of the target gene.

As used herein, the term "transgene" refers to any nucleic acid molecule, which is inserted by artifice into a cell, and becomes part of the genome of the organism that develops from the cell. Such a transgene may include a gene that is partly or entirely heterologous (*i*.*e*., foreign) to the transgenic organism, or may represent a gene homologous to an endogenous gene of the organism. The term "transgene" also means a nucleic acid molecule that includes one or more selected nucleic acid sequences, *e*.*g*., DNAs, that encode one or more engineered RNA precursors, to be expressed in a transgenic organism, *e*.*g*., animal, which is partly or entirely heterologous, *i*.*e*., foreign, to the transgenic animal, or homologous to an endogenous gene of the transgenic animal, but which is designed to be inserted into the animal's genome at a location which differs from that of the natural gene. A transgene includes one or more promoters and any other DNA, such as introns, necessary for expression of the selected nucleic acid sequence, all operably linked to the selected sequence, and may include an enhancer sequence.

A gene "involved" in a disease or disorder includes a gene, the normal or aberrant expression or function of which effects or causes the disease or disorder or at least one symptom of said disease or disorder

"Allele specific inhibition of expression" refers to the ability to significantly inhibit expression of one allele of a gene over another, *e*.*g*., when both alleles are present in the same cell. For example, the alleles can differ by one, two, three or more nucleotides. In some cases, one allele is associated with disease causation, *e*.*g*., a disease correlated to a dominant gain-of-function mutation.

The term "gain-of-function mutation" as used herein, refers to any mutation in a gene in which the protein encoded by said gene (*i*.*e*., the mutant protein) acquires a function not normally associated with the protein (*i*.*e*., the wild type protein) causes or contributes to a disease or disorder. The gain-of-function mutation can be a deletion, addition, or substitution of a nucleotide or nucleotides in the gene which gives rise to the change in the function of the encoded protein. In one embodiment, the gain-of-function mutation changes the function of the mutant protein or causes interactions with other proteins. In another embodiment, the gain-of-function mutation causes a decrease in or removal of normal wild-type protein, for example, by interaction of the altered, mutant protein with said normal, wild-type protein.

The phrase "examining the function of a gene in a cell or organism" refers to examining or studying the expression, activity, function or phenotype arising therefrom.

Various methodologies of the instant invention include step that involves comparing a value, level, feature, characteristic, property, etc. to a "suitable control", referred to interchangeably herein as an "appropriate control". A "suitable control" or "appropriate control" is any control or standard familiar to one of ordinary skill in the art useful for comparison purposes. In one embodiment, a "suitable control" or "appropriate control" is a value, level, feature, characteristic, property, etc. determined prior to performing an RNAi methodology, as described herein. For example, a transcription rate, mRNA level, translation rate, protein level, biological activity, cellular characteristic or property, genotype, phenotype, etc. can be determined prior to introducing an RNAi agent of the invention into a cell or organism. In another embodiment, a "suitable control" or "appropriate control" is a value, level, feature, characteristic, property, etc. determined in a cell or organism, *e*.*g*., a control or normal cell or organism, exhibiting, for example, normal traits. In yet another embodiment, a "suitable control" or "appropriate control" is a predefined value, level, feature, characteristic, property, etc.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

Various aspects of the invention are described in further detail in the following subsections.

### I. Amyotrophic lateral Sclerosis (ALS)

Amyotrophic lateral sclerosis (ALS), also known as Lou Gehrig's disease, is a progressive, fatal neurodegenerative disorder involving the motor neurons of the cortex, brain stem, and spinal cord (Hirano, A., 1996, Neurology 47 (Suppl. 2), S63-S66). The disease is caused by a dominant, gain-of-function mutation that develops in people bearing one mutant and one wild type copy of the gene *e*.*g*., SOD1. ALS causing SOD1 mutations are single-nucleotide point mutations that alter a single amino acid in the protein. The disease is further characterized by a progressive motor neuron degeneration leading to paralysis, to total loss of motor and respiratory functions, and eventually to death two to eight years after the appearance of the first clinical signs (mean duration after onset three years). ALS is of genetic origin in 10% of the patients, and sporadic in 90% of the cases. Point mutations in the gene encoding for copper zinc superoxide dismutase (SOD1) localized on chromosome 21q22-1 are responsible for the pathology in 20% of the familial cases (Rosen et al., Mutations in Cu/Zn superoxide dismutase gene are associated with familial amyotrophic lateral sclerosis, Nature, 362, 59-62, 1993, review in Rowland, Amyotrophic lateral sclerosis: Human challenge for neuroscience, Proc. Natl. Acad. Sci. USA, 92, 1251-1253, 1995). Thus, defective SOD1 is linked to motor neuron death and carries implications for understanding and possible treatment of familial amyotrophic lateral sclerosis.

### II. The SOD-1 gene

SOD1 is a metalloenzyme that contains one copper and one zinc, and is present in the cytoplasm as a homodimer. Copper is required for enzymatic activity while zinc stabilizes the protein's structure (Fridovich, 1986). SOD 1 is a expressed in all eukaryotic cells and is one of a family of three SOD enzymes, including manganese-dependent, mitochondrial SOD (SOD2) and copper/zinc extracellular SOD (SOD3) (I Fridovich, 1986, "Superoxide dismutases," Advances in Enzymology 58: 61-97). The main natural function of SOD1 is superoxide dismutation, in which superoxide (O₂⁻) is converted to hydrogen peroxide (H₂O₂) and oxygen. Together with the downstream enzymes catalase and glutathione peroxidase (which convert H₂O₂ to water and oxygen), SOD 1 detoxifies cellular free radicals. The importance of this function is underscored by numerous abnormalities in mice lacking the SOD1 gene, including reduced fertility (Matzuk et al., 1998), motor axonopathy (Shefner et al., 1999), increased age-associated loss of cochlear hair cells (McFadden et al., 2001) and neuromuscular junction synapses (Flood et al., 1999), and enhanced susceptibility to a variety of noxious assaults on the nervous system, such as axonal injury (Reaume et al., 1996), ischemia (Kondo et al., 1997; Kawase et al., 1999), hemolysate exposure (Matz et al., 2000) and irradiation (Behndig et al., 2001). Given the toxcicity of the mutant protein and the functional importance of the wild-type, the ideal therapy for ALS would be to selectively block expression of the mutant SOD1 protein while retaining expression of the wild-type SOD1 protein.

The present invention, targets mutant SOD1 using RNAi. The method utilized in RNAi comprises one strand of double-stranded RNA (siRNA) which complements a region containing a point mutation within the mutant SOD1 mRNA. After introduction of siRNA into neurons, the siRNA partially unwinds, binds to the region containing the point mutation within the SOD1 mRNA in a site-specific manner, and activates an mRNA nuclease. This nuclease cleaves the SOD1 mRNA, thereby halting translation of the mutant SOD1. Cells rid themselves of partially digested mRNA, thus precluding translation, or cells digest partially translated proteins. Neurons survive on the wild-type SOD1 (from the normal allele); this approach prevents the ravages of mutant SOD1 by eliminating its production.

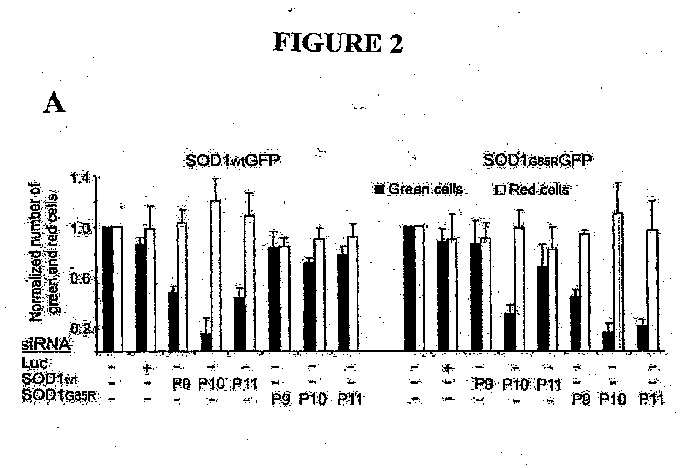

The amino acid sequence of human wild-type SOD1 protein is set forth in Figure 1 (SEQ ID NO:18). A consensus nucleotide sequence of human wild-type SOD1 gene (cDNA) is set forth in Figure 2 (SEQ ID NO:17)

### III. SOD-1 mutant gene

More than 100 SOD1 mutations have been identified. Most of these mutations produce a single amino acid replacement in the superoxide dismutase enzyme's chain of amino acids. The most common substitution, which occurs in 50 percent of American patients with type 1 amyotrophic lateral sclerosis, is the replacement of arginine with valine at position 4 in the amino acid chain (also written as Arg4Val).

SOD1 mutations affect the age when symptoms of type 1 amyotrophic lateral sclerosis begin and how fast the disease progresses. The Arg4Val mutation, for example, results in an aggressive form of the disorder with a survival time of less than 2 years after disease onset. The replacement of glycine with arginine at position 37 (Gly37Arg) is associated with early onset of the disease but a longer survival time. In addition, other factors in combination with SOD1 mutations probably vary the course of type 1 amyotrophic lateral sclerosis. For example, mutations in both the SOD1 gene and a gene known as CNTF appear to accelerate the onset of the disease. The CNTF mutation alone has no ill effects, but in combination with the SOD1 mutation, disease symptoms appear decades earlier compared to other affected family members.

It remains unclear how SOD1 mutations lead to the selective death of motor neurons, which are the specialized nerve cells in the brain and spinal cord that control muscle movement. The superoxide dismutase enzyme is thought to gain a new (but still undefined) toxic function as a result of changes in the SOD1 gene. The malfunctioning enzyme may cause the death of motor neurons through an accumulation of harmful superoxide radicals, abnormal production of other types of toxic radicals, promotion of cell suicide (apoptosis), clumping of the enzyme with other cell proteins, or continued stimulation of motor neurons that cause them to burn out and die (excitotoxicity).

**TABLE 1: SOD 1 mutations**

| Location | nt | aa | | | |
|---|---|---|---|---|---|
| exon 1 | 93 | 4 | Ala4Ser | Ala4Thr | Ala4Val |
| exon 1 | 99 | 6 | Cys6Gly | Cys6Phe | |
| exon 1 | 103 | 7 | Val7Glu | | |
| exon 1 | 105 | 8 | Leu8Val | Leu8Gln | |
| exon 1 | 112 | 10 | Gly10Val | Gly10Gly | |
| exon 1 | 117 | 12 | Gly12Arg | | |
| exon 1 | 123 | 14 | Val14Met | Val14Gly | |
| exon 1 | 129 | 16 | Gly16Ser | Gly16Ala | |
| exon 1 | 142 | 20 | Phe20Cys | | |
| exon 1 | 144 | 21 | Glu21Lys | Glu21Gly | |
| exon 1 | 148 | 22 | Gln22Leu | | |
| intron 1 | 319 | | 319t>a | | |
| exon 2 | 466 | 37 | Gyy37Arg | | |
| exon 2 | 469 | 38 | Leu38Val | Leu38Arg | |
| exon 2 | 478 | 41 | Gly41Ser | Gly41Asp | |
| exon 2 | 485 | 43 | His43Arg | | |
| exon 2 | 491 | 45 | Phe45Cys | | |
| exon 2 | 494 | 46 | His46Arg | | |
| exon 2 | 496 | 47 | Val47Phe | | |
| exon 2 | 500 | 48 | His48Arg | His48Gln | |
| exon 2 | 502 | 49 | Glu49Lys | | |
| exon 2 | 518 | 54 | Thr54Arg | | |
| exon 3 | 645 | 59 | Ser59Ile | Ser59Ser | |
| exon 3 | 663 | 65 | Asn65Ser | | |
| exon 3 | 669 | 67 | Leu67Arg | | |
| exon 3 | 683 | 72 | Gly72Cys | Gly72Ser | |
| exon 3 | 695 | 76 | Asp76Tyr | Asp76Val | |
| exon 4 | 1048 | 80 | His80Arg | | |
| exon 4 | 1059 | 84 | Leu84Val | Leu84Phe | |
| exon 4 | 1062 | 85 | Gly85Arg | | |
| exon 4 | 1066 | 86 | Asn86Ser | | |
| exon 4 | 1068 | 87 | Val87Met | Val87Ala | |
| exon 4 | 1071 | 88 | Thr88delACTGCT GAC | | |
| exon 4 | 1074 | 89 | Ala89Thr | Ala89Val | |
| exon 4 | 1078 | 90 | Asp90Ala | Asp90Val | |
| exon 4 | 1086 | 93 | Gly93Cys | Gly93Arg | Gly93Ser |
| | | | Gly93Asp | Gly93Ala | Gly93Val |
| exon 4 | 1092 | 95 | Ala95Thr | | |
| exon 4 | 1095 | 96 | Asp96Asn | | |
| exon 4 | 1098 | 97 | Val97Met | | |
| exon 4 | 1107 | 100 | Glu100Lys | Glu100Gly | |
| exon 4 | 1110 | 101 | Asp101Asn | Asp101Gly | |
| exon 4 | 1119 | 104 | Ile104Phe | | |
| exon 4 | 1122 | 105 | Ser105delTCACTC | Ser105Leu | |
| exon 4 | 1125 | 106 | Leu106Val | | |
| exon 4 | 1132 | 108 | Gly108Val | | |
| exon 4 | 1144 | 112 | Ile112Thr | Ile112Met | |
| exon 4 | 1146 | 113 | Ile113Phe | Ile113Thr | |
| exon 4 | 1150 | 114 | Gly114Ala | | |
| exon 4 | 1152 | 115 | Arg115Gly | | |
| exon 4 | 1161 | 118 | Val118Leu | Val118insA AAAC | |
| intron 4 | 1415 | | 1415t>g | | |
| exon 5 | 1441 | 124 | Asp124Gly | Asp124Val | |
| exon 5 | 1443 | 125 | Asp125His | | |
| exon 5 | 1446 | 126 | Leu26de1TT | Leu26STOP | Leu26Ser |
| exon 5 | 1450 | 127 | Gly127insTGGG | | |
| exon 5 | 1465 | 132 | Glu132insTT | | |
| exon 5 | 1467 | 133 | Glu133del | | |
| exon 5 | 1471 | 134 | Ser134Asn | | |
| exon 5 | 1487 | 139 | Asn139Asn | Asn139Lys | |
| exon 5 | 1489 | 140 | Ala140Gly | Ala140Ala | |
| exon 5 | 1491 | 141 | Gly141STOP | | |
| exon 5 | 1501 | 144 | Leu144Ser | Leu144Phe | |
| exon 5 | 1503 | 145 | Ala145Thr | Ala145Gly | |
| exon 5 | 1506 | 146 | Cys146Arg | | |
| exon 5 | 1509 | 147 | Gly147Arg | | |
| exon 5 | 1512 | 148 | Val148Ile | Val148Gly | |
| exon 5 | 1516 | 149 | Ile149Thr | | |
| exon 5 | 1522 | 151 | Ile151Thr | Ile151Ser | |
| exon 5 | 1529 | 153 | Gln153Gln | | |

### IV. RNA Interference

RNAi is a remarkably efficient process whereby double-stranded RNA (dsRNA) induces the sequence-specific degradation of homologous mRNA in animals and plant cells (Hutvagner and Zamore (2002), Curr. Opin. Genet. Dev., 12, 225-232; Sharp (2001), Genes Dev., 15, 485-490). In mammalian cells, RNAi can be triggered by 21-nucleotide (nt) duplexes of small interfering RNA (siRNA) (Chiu et al. (2002), Mol. Cell., 10, 549-561; Elbashir et al. (2001), Nature, 411, 494-498), or by micro-RNAs (miRNA), functional small-hairpin RNA (shRNA), or other dsRNAs which are expressed *in-vivo* using DNA templates with RNA polymerase III promoters (Zeng et al. (2002), Mol. Cell, 9, 1327-1333; Paddison et al. (2002), Genes Dev., 16, 948-958; Lee et al. (2002), Nature Biotechnol., 20, 500-505; Paul et al. (2002), Nature Biotechnol., 20, 505-508; Tuschl, T. (2002), Nature Biotechnol., 20, 440-448; Yu et al. (2002), Proc. Natl. Acad. Sci. USA, 99(9), 6047-6052; McManus et al. (2002), RNA, 8, 842-850; Sui et al. (2002), Proc. Natl. Acad. Sci. USA, 99(6), 5515-5520.)

### V. RNA Molecules and Agents

The present invention features "small interfering RNA molecules" ("siRNA molecules" or "siRNA"), methods of making said siRNA molecules and methods (*e*.*g*., research and/or therapeutic methods) for using said siRNA molecules. An siRNA molecule of the invention is a duplex consisting of a sense strand and complementary antisense strand, the antisense strand having sufficient complementary to a target mRNA to mediate RNAi. Preferably, the strands are aligned such that there are at least 1, 2, or 3 bases at the end of the strands which do not align (*i.e.*, for which no complementary bases occur in the opposing strand) such that an overhang of 1, 2 or 3 residues occurs at one or both ends of the duplex when strands are annealed. Preferably, the siRNA molecule has a length from about 10-50 or more nucleotides, *i*.*e*., each strand comprises 10-50 nucleotides (or nucleotide analogs). More preferably, the siRNA molecule has a length from about 16 -30, *e*.*g*., 16, 17, 18, 19, 20, 21, 22, 23, 24, 25, 26, 27, 28, 29, or 30 nucleotides in each strand, wherein one of the strands is substantially complementary to, *e*.*g*., at least 80% (or more, *e*.*g*., 85%, 90%, 95%, or 100%) complementary to, e.g., having 3, 2, 1, or 0 mismatched nucleotide(s), a target region, such as a target region that differs by at least one base pair between the wild type and mutant allele, e.g., a target region comprising the gain-of-function mutation, and the other strand is identical or substantially identical to the first strand.

Generally, siRNAs can be designed by using any method known in the art, for instance, by using the following protocol:
1. Beginning with the AUG start codon of, look for AA dinucleotide sequences; each AA and the 3' adjacent 16 or more nucleotides are potential siRNA targets. The siRNA should be specific for a target region that differs by at least one base pair between the wild type and mutant allele, *e*.*g*., a target region comprising the gain of function mutation. The first strand should be complementary to this sequence, and the other strand is identical or substantially identical to the first strand. In one embodiment, the nucleic acid molecules are selected from a region of the target allele sequence beginning at least 50 to 100 nt downstream of the start codon, *e*.*g*., of the sequence of SOD 1. Further, siRNAs with lower G/C content (35-55%) may be more active than those with G/C content higher than 55%. Thus in one embodiment, the invention includes nucleic acid molecules having 35-55% G/C content. In addition, the strands of the siRNA can be paired in such a way as to have a 3' overhang of 1 to 4, *e*.*g*., 2, nucleotides. Thus in another embodiment, the nucleic acid molecules may have a 3' overhang of 2 nucleotides, such as TT. The overhanging nucleotides may be either RNA or DNA. As noted above, it is desirable to choose a target region wherein the mutant:wild type mismatch is a purine:purine mismatch.
2. Using any method known in the art, compare the potential targets to the appropriate genome database (human, mouse, rat, etc.) and eliminate from consideration any target sequences with significant homology to other coding sequences. One such method for such sequence homology searches is known as BLAST, which is available at National Center for Biotechnology Information website.
3. Select one or more sequences that meet your criteria for evaluation.
Further general information about the design and use of siRNA may be found in "The siRNA User Guide," available at The Max-Plank-Institut für Biophysikalishe Chemie website.

Negative control siRNAs should have the same nucleotide composition as the selected siRNA, but without significant sequence complementarity to the appropriate genome. Such negative controls may be designed by randomly scrambling the nucleotide sequence of the selected siRNA; a homology search can be performed to ensure that the negative control lacks homology to any other gene in the appropriate genome. In addition, negative control siRNAs can be designed by introducing one or more base mismatches into the sequence. siRNA's having single nucleotide specificity can be designed as follows:

A target mRNA is selected (*e*.*g*., a mutant allele or mRNA) having a mismatch (*e*.*g*., a single nucleotide mismatch, for example a point mutation) as compared to a reference mRNA sequence (*e*.*g*., a wild type allele or mRNA sequence). siRNAs are designed such that perfect complementarity exists between the siRNA and the target mRNA (*e*.*g*., the mutant mRNA) at the single nucleotide (*e*.*g*., the point mutation), there thus being a mismatch if the siRNA is compared (*e*.*g*., aligned) to the reference sequence (*e*.*g*., wild type allele or mRNA sequence). Preferably the siRNA is designed such that the single nucleotide (*e*.*g*., the point mutation) is at or near the intended site of cleavage. Preferably, the siRNA is designed such that single nucleotide (*e*.*g*., the point mutation) being targeted is perfectly or exactly centered in the siRNA (*e*.*g*., in the antisense strand of the siRNA). The phrase perfectly centered means that there are the same number of nucleotides flanking (*i*.*e*., 8, 9, 10, 11 or 12) the single nucleotide (*e*.*g*., the point mutation), but for any overhang, for example, a dTdT tail. For example, if a 21-nucleotide siRNA is chosen having a 2-nucleotide 3' overhang (*e*.*g*., overhang at the 3' end of the antisense strand), there are 9 nucleotides flanking the single nucleotide (*e*.*g*., point mutation). For a 22-nucleotide siRNA having a 2-nucleotide 3' overhang (*e*.*g*., overhang at the 3' end of the antisense strand) there are 9 and 10 nucleotides flanking the single nucleotide (*e*.*g*., point mutation). For a 23-nucleotide siRNA, there are 10 nucleotides flanking the single nucleotide (*e*.*g*., point mutation). For a 24-nucleotide siRNA, there are 10 and 11 nucleotides flanking the single nucleotide (*e*.*g*., point mutation). The numbers exemplified are for siRNAs having 2-nucleotide 3' overhangs but can be readily adjusted for siRNAs having longer or shorter overhangs or no overhangs. Designing the siRNA such that the single nucleotide (*e*.*g*., point mutation is off-center with respect to the siRNA may, in some instances, reduce efficiency of cleavage by the siRNA.

siRNAs with single nucleotide specificity are preferably designed such that base paring at the single nucleotide in the corresponding reference (*e*.*g*., wild type) sequence is disfavored. For example, designing the siRNA such that purine:purine paring exists between the siRNA and the wild type mRNA at the single nucleotide enhances single nucleotide specificity. The purine:purine paring is selected, for example, from the group G:G, A:G, G:A and A:A pairing. Moreover, purine pyrimidine pairing between the siRNA and the mutant mRNA at the single nucleotide enhances single nucleotide specificity. The purine:pyrimidine paring is selected, for example, from the group G:C, C:G, A:U, U:A, C:A, A:C, U:A and A:U pairing.

The nucleic acid compositions of the invention include both siRNA and siRNA derivatives as described herein. For example, cross-linking can be employed to alter the pharmacokinetics of the composition, for example, to increase half-life in the body. Thus, the invention includes siRNA derivatives that include siRNA having two complementary strands of nucleic acid, such that the two strands are crosslinked. The invention also includes siRNA derivatives having a non-nucleic acid moiety conjugated to its 3' terminus (*e*.*g*., a peptide), organic compounds (*e*.*g*., a dye), or the like. Modifying siRNA derivatives in this way may improve cellular uptake or enhance cellular targeting activities of the resulting siRNA derivative as compared to the corresponding siRNA, are useful for tracing the siRNA derivative in the cell, or improve the stability of the siRNA derivative compared to the corresponding siRNA.

The siRNA molecules of the invention can be chemically synthesized, or can be transcribed *in-vitro* from a DNA template, or *in-vivo* from *e*.*g*., shRNA, or, by using recombinant human DICER enzyme, to cleave *in-vitro* transcribed dsRNA templates into pools of 20- ,21- or 23- bp duplex RNA mediating RNAi. The siRNA molecules can be designed using any method known in the art.

In one aspect, instead of the RNAi agent being an interfering ribonucleic acid, *e*.*g*., an siRNA or shRNA as described above, the RNAi agent can encode an interfering ribonucleic acid, *e*.*g*., an shRNA, as described above. In other words, the RNAi agent can be a transcriptional template of the interfering ribonucleic acid. Thus, RNAi agents of the present invention can also include small hairpin RNAs (shRNAs), and expression constructs engineered to express shRNAs. Transcription of shRNAs is initiated at a polymerase III (pol III) promoter, and is thought to be terminated at position 2 of a 4-5-thymine transcription termination site. Upon expression, shRNAs are thought to fold into a stem-loop structure with 3' UU-overhangs; subsequently, the ends of these shRNAs are processed, converting the shRNAs into siRNA-like molecules of about 21-23 nucleotides. Brummelkamp et al., Science 296:550-553 (2002); Lee et al, (2002). *supra*; Miyagishi and Taira, Nature Biotechnol. 20:497-500 (2002); Paddison *et al.* (2002), *supra*; Paul (2002), *supra*; Sui (2002) *supra*; Yu *et al.* (2002), *supra.*

Expression constructs of the present invention include any construct suitable for use in the appropriate expression system and include, but are not limited to, retroviral vectors, linear expression cassettes, plasmids and viral or virally-derived vectors, as known in the art. Such expression constructs can include one or more inducible promoters, RNA Pol III promoter systems such as U6 snRNA promoters or H1 RNA polymerase III promoters, or other promoters known in the art. The constructs can include one or both strands of the siRNA. Expression constructs expressing both strands can also include loop structures linking both strands, or each strand can be separately transcribed from separate promoters within the same construct. Each strand can also be transcribed from a separate expression construct. (Tuschl (2002), *supra*).

Synthetic siRNAs can be delivered into cells by methods known in the art, including cationic liposome transfection and electroporation. However, these exogenous siRNA generally show short term persistence of the silencing effect (4∼5 days in cultured cells), which may be beneficial in only certain embodiments. To obtain longer term suppression of the target genes (*i*.*e*., mutant genes) and to facilitate delivery under certain circumstances, one or more siRNA can be expressed within cells from recombinant DNA constructs. Such methods for expressing siRNA duplexes within cells from recombinant DNA constructs to allow longer-term target gene suppression in cells are known in the art, including mammalian Pol III promoter systems (*e*.*g*., H1 or U6/snRNA promoter systems (Tuschl (2002), *supra*) capable of expressing functional double-stranded siRNAs; (Bagella et al., J. Cell. Physiol. 177:206-213 (1998); Lee *et al.* (2002), *supra*; Miyagishi *et al.* (2002), *supra*; Paul *et al.* (2002), *supra*; Yu *et al.* (2002), *supra*; Sui *et al.* (2002), *supra*). Transcriptional termination by RNA Pol III occurs at runs of four consecutive T residues in the DNA template, providing a mechanism to end the siRNA transcript at a specific sequence. The siRNA is complementary to the sequence of the target gene in 5'-3' and 3'-5' orientations, and the two strands of the siRNA can be expressed in the same construct or in separate constructs. Hairpin siRNAs, driven by H1 or U6 snRNA promoter and expressed in cells, can inhibit target gene expression (Bagella *et al*. (1998), *supra;* Lee *et al.* (2002), *supra*; Miyagishi *et al.* (2002), *supra;* Paul *et al.* (2002), *supra*; Yu *et al.* (2002), *supra*; Sui *et al.* (2002) *supra*). Constructs containing siRNA sequence under the control of T7 promoter also make functional siRNAs when cotransfected into the cells with a vector expressing T7 RNA polymerase (Jacque (2002), *supra*). A single construct may contain multiple sequences coding for siRNAs, such as multiple regions of the gene encoding mutant SOD1, targeting the same gene or multiple genes, and can be driven, for example, by separate PolIII promoter sites.

Animal cells express a range of noncoding RNAs of approximately 22 nucleotides termed micro RNA (miRNAs) which can regulate gene expression at the post transcriptional or translational level during animal development. One common feature of miRNAs is that they are all excised from an approximately 70 nucleotide precursor RNA stem-loop, probably by Dicer, an RNase III-type enzyme, or a homolog thereof. By substituting the stem sequences of the miRNA precursor with sequence complementary to the target mRNA, a vector construct that expresses the engineered precursor can be used to produce siRNAs to initiate RNAi against specific mRNA targets in mammalian cells (Zeng (2002), *supra*). When expressed by DNA vectors containing polymerase III promoters, micro-RNA designed hairpins can silence gene expression (McManus (2002), *supra*). MicroRNAs targeting polymorphisms may also be useful for blocking translation of mutant proteins, in the absence of siRNA-mediated gene-silencing. Such applications may be useful in situations, for example, where a designed siRNA caused off-target silencing of wild type protein.

Viral-mediated delivery mechanisms can also be used to induce specific silencing of targeted genes through expression of siRNA, for example, by generating recombinant adenoviruses harboring siRNA under RNA Pol II promoter transcription control (Xia *et al.* (2002), *supra*). Infection of HeLa cells by these recombinant adenoviruses allows for diminished endogenous target gene expression. Injection of the recombinant adenovirus vectors into transgenic mice expressing the target genes of the siRNA results in *in-vivo* reduction of target gene expression. *Id*. In an animal model, whole-embryo electroporation can efficiently deliver synthetic siRNA into post-implantation mouse embryos (Calegari et al., Proc. Natl. Acad. Sci. USA 99(22):14236-40 (2002)). In adult mice, efficient delivery of siRNA can be accomplished by "highpressure" delivery technique, a rapid injection (within 5 seconds) of a large volume of siRNA containing solution into animal *via* the tail vein (Liu (1999), *supra;* McCaffrey (2002), *supra*; Lewis, Nature Genetics 32:107-108 (2002)). Nanoparticles and liposomes can also be used to deliver siRNA into animals.

The nucleic acid compositions of the invention include both unmodified siRNAs and modified siRNAs as known in the art, such as crosslinked siRNA derivatives or derivatives having non nucleotide moieties linked, for example to their 3' or 5' ends. Modifying siRNA derivatives in this way may improve cellular uptake or enhance cellular targeting activities of the resulting siRNA derivative as compared to the corresponding siRNA, are useful for tracing the siRNA derivative in the cell, or improve the stability of the siRNA derivative compared to the corresponding siRNA.

Engineered RNA precursors, introduced into cells or whole organisms as described herein, will lead to the production of a desired siRNA molecule. Such an siRNA molecule will then associate with endogenous protein components of the RNAi pathway to bind to and target a specific mRNA sequence for cleavage and destruction. In this fashion, the mRNA to be targeted by the siRNA generated from the engineered RNA precursor will be depleted from the cell or organism, leading to a decrease in the concentration of the protein encoded by that mRNA in the cell or organism. The RNA precursors are typically nucleic acid molecules that individually encode either one strand of a dsRNA or encode the entire nucleotide sequence of an RNA hairpin loop structure.

The nucleic acid compositions of the invention can be unconjugated or can be conjugated to another moiety, such as a nanoparticle, to enhance a property of the compositions, *e*.*g*., a pharmacokinetic parameter such as absorption, efficacy, bioavailability, and/or half-life. The conjugation can be accomplished by methods known in the art, e.g., using the methods of Lambert et al., Drug Deliv. Rev.:47(1), 99-112 (2001) (describes nucleic acids loaded to polyalkylcyanoacrylate (PACA) nanoparticles); Fattal et al., J. Control Release 53(1-3):137-43 (1998) (describes nucleic acids bound to nanoparticles); Schwab et al., Ann. Oncol. 5 Suppl. 4:55-8 (1994) (describes nucleic acids linked to intercalating agents, hydrophobic groups, polycations or PACA nanoparticles); and Godard et al., Eur. J. Biochem. 232(2):404-10 (1995) (describes nucleic acids linked to nanoparticles).

The nucleic acid molecules of the present invention can also be labeled using any method known in the art; for instance, the nucleic acid compositions can be labeled with a fluorophore, *e*.*g*., Cy3, fluorescein, or rhodamine. The labeling can be carried out using a kit, e.g., the SILENCER™ siRNA labeling kit (Ambion). Additionally, the siRNA can be radiolabeled, *e*.*g*., using ³H, ³²P, or other appropriate isotope.

Moreover, because RNAi is believed to progress *via* at least one single-stranded RNA intermediate, the skilled artisan will appreciate that ss-siRNAs (*e*.*g*., the antisense strand of a ds-siRNA) can also be designed (*e*.*g*., for chemical synthesis) generated (*e*.*g*., enzymatically generated)or expressed (*e*.*g*., from a vector or plasmid) as described herein and utilized according to the claimed methodologies. Moreover, in invertebrates, RNAi can be triggered effectively by long dsRNAs (*e*.*g*., dsRNAs about 100 - 1000 nucleotides in length, preferably about 200- 500, for example, about 250, 300, 350, 400 or 450 nucleotides in length) acting as effectors of RNAi. (Brondani et al., Proc Natl Acad Sci U S A. 2001 Dec 4;98(25):14428-33. Epub 2001 Nov 27).

The siRNA molecules of the present invention can comprise or consists of the sequences as listed in Figure 1A including mutant siRNAP11 (SEQ ID NO: 5, sense; SEQ ID NO: 6, anti-sense or guide), mutant siRNAP10 (SEQ ID NO: 3, sense; SEQ ID NO: 4, anti-sense or guide), mutant siRNA P9 (SEQ ID NO: 1, sense; SEQ ID NO: 2 anti-sense or guide), SOD1 wild-type target (SEQ ID NO: 7), SOD1 mutant target (SEQ ID NO: 8), wild-type siRNAP11 (SEQ ID NO: 9 sense; SEQ ID NO: 10, anti-sense or guide), wild-type siRNAP10 (SEQ ID NO: 11, sense; SEQ ID NO: 12, anti-sense or guide), wild-type siRNA P9 (SEQ ID NO: 13, sense; SEQ ID NO: 14, anti-sense or guide); Figure 3A including G93A SOD1 siRNA (SEQ ID NO:16), and allelic variants thereof.

### VI. Uses of Engineered RNA Precursors to Induce RNAi

Engineered RNA precursors, introduced into cells or whole organisms as described herein, will lead to the production of a desired siRNA molecule. Such an siRNA molecule will then associate with endogenous protein components of the RNAi pathway to bind to and target a specific mRNA sequence for cleavage and destruction. In this fashion, the mRNA to be targeted by the siRNA generated from the engineered RNA precursor will be depleted from the cell or organism, leading to a decrease in the concentration of the protein encoded by that mRNA in the cell or organism.

### VII. Pharmaceutical Compositions and Methods of Administration

The siRNA molecules of the invention can be incorporated into pharmaceutical compositions. Such compositions typically include the nucleic acid molecule and a pharmaceutically acceptable carrier. As used herein the language "pharmaceutically acceptable carrier" includes saline, solvents, dispersion media, coatings, antibacterial and antifungal agents, isotonic and absorption delaying agents, and the like, compatible with pharmaceutical administration. Supplementary active compounds can also be incorporated into the compositions.

A pharmaceutical composition is formulated to be compatible with its intended route of administration. Examples of routes of administration include parenteral, *e*.*g*., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle, which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, *e*.*g*., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, *e*.*g*., a gas such as carbon dioxide, or a nebulizer. Such methods include those described in U.S. Patent No. 6,468,798.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (*e*.*g*., with conventional suppository bases such as cocoa butter and other glycerides) or retention enemas for rectal delivery.

The compounds can also be administered by transfection or infection using methods known in the art, including but not limited to the methods described in McCaffrey et al. (2002), Nature, 418(6893), 38-9 (hydrodynamic transfection); Xia et al. (2002), Nature Biotechnol., 20(10), 1006-10 (viral-mediated delivery); or Putnam (1996), Am. J. Health Syst. Pharm. 53(2), 151-160, erratum at Am. J. Health Syst. Pharm. 53(3), 325 (1996).

The compounds can also be administered by any method suitable for administration of nucleic acid agents, such as a DNA vaccine. These methods include gene guns, bio injectors, and skin patches as well as needle-free methods such as the micro-particle DNA vaccine technology disclosed in U.S. Patent No. 6,194,389, and the mammalian transdermal needle-free vaccination with powder-form vaccine as disclosed in U.S. Patent No. 6,168,587. Additionally, intranasal delivery is possible, as described in, *inter alia*, Hamajima et al. (1998), Clin. Immunol. Immunopathol., 88(2), 205-10. Liposomes (*e*.*g*., as described in U.S. Patent No. 6,472,375) and microencapsulation can also be used. Biodegradable targetable microparticle delivery systems can also be used (*e*.*g*., as described in U.S. Patent No. 6,471,996).

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Such formulations can be prepared using standard techniques. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

Toxicity and therapeutic efficacy of such compounds can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds which exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC50 (*i*.*e*., the concentration of the test compound which achieves a half-maximal inhibition of symptoms) as determined in cell culture. Such information can be used to more accurately determine useful doses in humans. Levels in plasma may be measured, for example, by high performance liquid chromatography.

As defined herein, a therapeutically effective amount of a nucleic acid molecule (*i*.*e*., an effective dosage) depends on the nucleic acid selected. For instance, if a plasmid encoding shRNA is selected, single dose amounts in the range of approximately 1 :g to 1000 mg may be administered; in some embodiments, 10, 30, 100 or 1000 :g may be administered. In some embodiments, 1-5 g of the compositions can be administered. The compositions can be administered one from one or more times per day to one or more times per week; including once every other day. The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of a protein, polypeptide, or antibody can include a single treatment or, preferably, can include a series of treatments.

The nucleic acid molecules of the invention can be inserted into expression constructs, *e*.*g*., viral vectors, retroviral vectors, expression cassettes, or plasmid viral vectors, *e*.*g*., using methods known in the art, including but not limited to those described in Xia et al., (2002), *supra.* Expression constructs can be delivered to a subject by, for example, inhalation, orally, intravenous injection, local administration (see U.S. Patent 5,328,470) or by stereotactic injection (see e.g., Chen et al. (1994), Proc. Natl. Acad. Sci. USA, 91, 3054-3057). The pharmaceutical preparation of the delivery vector can include the vector in an acceptable diluent, or can comprise a slow release matrix in which the delivery vehicle is imbedded. Alternatively, where the complete delivery vector can be produced intact from recombinant cells, e.g., retroviral vectors, the pharmaceutical preparation can include one or more cells which produce the gene delivery system.

The nucleic acid molecules of the invention can also include small hairpin RNAs (shRNAs), and expression constructs engineered to express shRNAs. Transcription of shRNAs is initiated at a polymerase III (pol III) promoter, and is thought to be terminated at position 2 of a 4-5-thymine transcription termination site. Upon expression, shRNAs are thought to fold into a stem-loop structure with 3' UU-overhangs; subsequently, the ends of these shRNAs are processed, converting the shRNAs into siRNA-like molecules of about 21 nucleotides. Brummelkamp et al. (2002), Science, 296, 550-553; Lee et al, (2002). *supra*; Miyagishi and Taira (2002), Nature Biotechnol., 20, 497-500; Paddison et al. (2002), *supra;* Paul (2002), *supra*; Sui (2002) *supra*; Yu et al. (2002), *supra*.

The expression constructs may be any construct suitable for use in the appropriate expression system and include, but are not limited to retroviral vectors, linear expression cassettes, plasmids and viral or virally-derived vectors, as known in the art. Such expression constructs may include one or more inducible promoters, RNA Pol III promoter systems such as U6 snRNA promoters or H1 RNA polymerase III promoters, or other promoters known in the art. The constructs can include one or both strands of the siRNA. Expression constructs expressing both strands can also include loop structures linking both strands, or each strand can be separately transcribed from separate promoters within the same construct. Each strand can also be transcribed from a separate expression construct, Tuschl (2002), *supra*.

The pharmaceutical compositions can be included in a container, pack, or dispenser together with instructions for administration.

### VIII. Methods of Treatment

The present invention provides for both prophylactic and therapeutic methods of treating a subject at risk of (or susceptible to) a disease or disorder caused, in whole or in part, by a gain- of-function mutant protein. In one embodiment, the disease or disorder is a dominant gain-or-function disease. In a preferred embodiment, the disease or disorder is a disorder associated with the an alteration of SOD 1 gene, specifically a point mutation in the SOD1 mutant allele, leading to a defect in SOD 1 gene (structure or function) or SOD1 protein (structure or function or expression), such that clinical manifestations include those seen in ALS disease patients.

"Treatment", or "treating" as used herein, is defined as the application or administration of a therapeutic agent (*e.g*., a RNA agent or vector or trans gene encoding same) to a patient, or application or administration of a therapeutic agent to an isolated tissue or cell line from a patient, who has the disease or disorder, a symptom of disease or disorder or a predisposition toward a disease or disorder, with the purpose to cure, heal, alleviate, relieve, alter, remedy, ameliorate, improve or affect the disease or disorder, the symptoms of the disease or disorder, or the predisposition toward disease.

In one aspect, the invention provides a method for preventing in a subject, a disease or disorder as described above, by administering to the subject a therapeutic agent (*e.g.*, a RNAi agent or vector or transgene encoding same). Subjects at risk for the disease can be identified by, for example, any or a combination of diagnostic or prognostic assays as described herein. Administration of a prophylactic agent can occur prior to the manifestation of symptoms characteristic of the disease or disorder, such that the disease or disorder is prevented or, alternatively, delayed in its progression.

Another aspect of the invention pertains to methods treating subjects therapeutically, i.e., alter onset of symptoms of the disease or disorder. In an exemplary embodiment, the modulatory method of the invention involves contacting a cell expressing a gain-of-function mutant with a therapeutic agent (*e.g.*, a RNAi agent or vector or transgene encoding same) that is specific for a mutation within the gene, such that sequence specific interference with the gene is achieved. These methods can be performed *in vitro* (*e.g.*, by culturing the cell with the agent) or, alternatively, *in vivo* (*e.g.*, by administering the agent to a subject).

With regards to both prophylactic and therapeutic methods of treatment, such treatments may be specifically tailored or modified, based on knowledge obtained from the field of pharmacogenomics. "Pharmacogenomics", as used herein, refers to the application of genomics technologies such as gene sequencing, statistical genetics, and gene expression analysis to drugs in clinical development and on the market. More specifically, the term refers the study of how a patient's genes determine his or her response to a drug (*e.g.*, a patient's "drug response phenotype", or "drug response genotype"). Thus, another aspect of the invention provides methods for tailoring an individual's prophylactic or therapeutic treatment with either the target gene molecules of the present invention or target gene modulators according to that individual's drug response genotype. Pharmacogenomics allows a clinician or physician to target prophylactic or therapeutic treatments to patients who will most benefit from the treatment and to avoid treatment of patients who will experience toxic drug-related side effects.

Therapeutic agents can be tested in an appropriate animal model. For example, an RNAi agent (or expression vector or transgene encoding same) as described herein can be used in an animal model to determine the efficacy, toxicity, or side effects of treatment with said agent. Alternatively, a therapeutic agent can be used in an animal model to determine the mechanism of action of such an agent. For example, an agent can be used in an animal model to determine the efficacy, toxicity, or side effects of treatment with such an agent. Alternatively, an agent can be used in an animal model to determine the mechanism of action of such an agent.

### EXAMPLES

The following materials, methods, and examples are illustrative only and not intended to be limiting.

### Materials And Methods

### RNA and DNA constructs

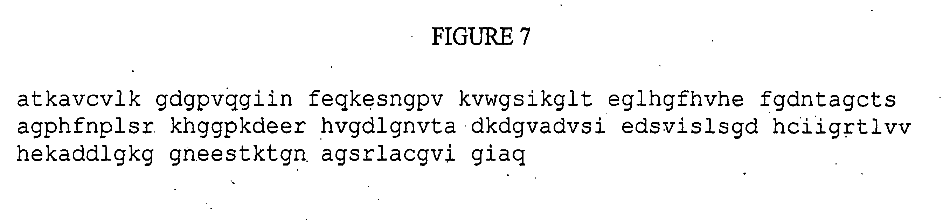

Twenty one nucleotide single strand RNAs (Fig. 1A) were purchased from Dharmacon Research, deprotected according to manufacturer's instructions, and annealed as described (Nykanen et al., 2001). To create wild type and mutant SOD1-GFP fusion proteins, SOD1wt (Genbank Accession No. NP_000445; Figure 7; SEQ ID NO: 18), SOD1 G85R and SOD1 G93A (SEQ ID NO:16) cDNAs were PCR cloned between the Pm1I and PstI sites of pCMV/myc/mito/GFP (Invitrogen). This cloning step deleted the mitochondrial targeting sequence. To create myc tagged wild type SOD1, SODlwt cDNA (SEQ ID NO:17) was PCR cloned between the PstI and XhoI sites of pCMV/myc/mito/GFP. The mitochondrial targeting sequence was then deleted by digestion with BssHII and PmlI and blunt ligation. All constructs were verified by sequencing. DsRed (pDsRed2-C1) was purchased from Clontech (Palo Alto, CA). U6-G93A was constructed as described (Sui et al., 2002). The 3'-block siRNA was synthesized by standard techniques.

### In-vitro RNAi assay

Five hundred and sixty nucleotide human SOD1 target RNAs containing either wild-type or mutant SOD1 coding sequence were prepared as described previously (Zamore et al., 2000). Target cleavage was determined by incubating a ∼5 nM concentration of the 5', ³²P-cap-radiolabeled target RNA with 25-100 nM siRNA in a standard *in-vitro* RNAi reaction containing *Drosophila* embryo lysate (Tuschl et al., 1999; Zamore et al., 2000).

### Cell culture and transfection

Hela cells were cultured in DMEM and N2A cells in DMED and Opti-MEM (1:1), both supplemented with 10% fetal bovine serum (FBS), 100 units ml⁻¹ penicillin, and 100 ug ml⁻¹ streptomycin. Twenty-four hours before transfection, cells (70-90% confluency) detached by trituration, transfered to 6-well plates and cultured in fresh medium without antibiotics. Transfection was carried out using lipofectamine® 2000 (Invitrogen) according to manufacturer's instructions. The amount of the constructs used in transfections are 4 µg each of mutant or wild type SOD1-GFP and DsRed plasmids, 4x10⁻¹¹ or 4x10⁻¹² mole siRNAs, and 20 or 8 µg U6-G93A.

### In-vivo transfection

Twenty-four mice 6-8 weeks old were divided into three groups. The first group received no shRNA vector, the second group received 20 µg empty vector and the third group received 20 µg U6-hpRNA vector against SOD1 G93A (SEQ ID NO:16). All groups received both 20 µg of myc tagged human wild type SOD1 (SEQ ID NO: 7) and 20 µg GFP tagged SOD1. The vectors were diluted in Ringer's solution so that the total volume equaled 2.5ml per mouse. Mice were anaesthetized with avertin (240mg/kg) and the vectors were injected into the tail vein using a 26-gauge needle in less than 10 seconds. Forty-eight hours following injection animals were perfused with 5ml PBS in order to remove blood from the liver. Livers were dissected and quickly frozen on dry ice. Samples were placed in 25mM PBS buffer (pH 7.2) containing 1% SDS, 1 mM DTT, 1 mM phenylmethylsufonyl fluoride (PMSF), and protease inhibitor cocktail (Sigma, diluted 1:100) and homogenized using a hand held polytrone (Pro-scientific).

### Western blot analysis

Protein concentrations were determined using a BCA protein assay kit (Pierce; Rockville IL). Twenty five µg Hela cell proteins or 100 µg liver proteins were separated on a 15% SDS-PAGE gel and transferred onto Genescreen Plus membrane (Perkin Elmer). Rabbit anti-SOD1 (Biodesign) or Sheep anti-SOD1 was the primary and HRP-labeled goat anti-rabbit IgG (Amersham) or donkey anti-sheep IgG was the secondary antibodies. The protein bands were visualized using SuperSignal kit (Pierce) and Kodak Digital Image Station 440CF. The intensity of the bands was quantified using Kodak 1D software.

### EXAMPLE I:

Examples I- VIII show that siRNAs were designed to have single-nucleotide selectivity by first testing siRNA activity in a cell-free RNAi reaction containing *Drosophila* embryo lysate, then analyzing active, single-nucleotide-selective siRNAs in cultured mammalian cells. Results showed that both 21 nucleotide siRNAs and shRNA can be designed that selectively inhibit the expression of the mutant (SEQ ID NO:8), but not of the wild type SOD1 (SEQ ID NO:7), even though the two mRNAs differ by only a single nucleotide and are present in the same cells. Thus, RNAi is useful as a therapy for diseases caused by dominant, gain-of-function type of mutations, *inter alia*.

### EXAMPLE I: siRNA duplexes can discriminate for mutant SOD1

Two sets of three siRNAs, each targeting either wild type or mutant SOD1 mRNA (Fig. 1A; SEQ ID NO:8) were designed to test whether mismatches at or near the site of target cleavage would disrupt the required A-form Helix. An allele of SOD1 in which guanosine 256 (G256; relative to the start of translation, e.g., of Genbank Accession No. K00056:) is mutated to cytosine, generating a glycine-to-arginine mutation (G85R) was selected. The mutated nucleotide was positioned near the predicted site of SOD1 mRNA cleavage, *i.e*., position 9 (P9), 10 (P10), or 11 (P11) relative to the 5' end of the antisense strand of the siRNA (Fig. 1A). This predicted site of SOD1 mRNA cleavage would place a mismatch between the siRNA and its non-cognate target RNA in or near the active site of the RNAi endonuclease. These siRNAs were tested in an established *Drosophila* embryo lysate reaction that recapitulates RNA *in- vitro* (Zamore et al., 2000; Tuschl et al., 1999). As expected, each of the six siRNAs cleaved the corresponding target RNA, although with dramatically different efficacy. For example, the P11 mutant and wild type siRNAs (SEQ ID NO: 6, 10) did not cut their respective target RNA efficiently. On the other hand, the P10 mutant siRNA (SEQ ID NO:4) efficiently cleaved the mutant target RNA. The destruction of the full-length mutant SOD 1 target mRNA was accompanied by a corresponding accumulation of 5' cleavage product of approximately 288 nucleotides, a result indicative ofRNAi, rather than non-specific degradation of the target mRNA. In the absence of siRNA or in the presence of an siRNA against the luciferase, the mutant SOD1 target RNA (SEQ ID NO:8) was stable in the *Drosophila* embryo lysate (data not shown). Data for both the destruction of target RNA and the accumulation of 5' cleavage product fit well to a single exponential equation, indicating that the reaction follows pseudo first-order kinetics (Fig. 1B).

### EXAMPLE I: siRNA duplexes can discriminate for wild-type SOD1

To determine the specificity of the six siRNAs, each siRNA corresponding to the mutant SOD1 sequence (SEQ ID NO:8) was tested for its ability to cleave the wild-type SOD1 RNA (SEQ ID NO:7), and each wild-type siRNA was tested for its ability to cleave mutant RNA. Some, but not all of the siRNA duplexes effectively discriminated between the target to which they are matched completely and the target with which they have a single nucleotide mismatch (Fig 1A). For example, P11 of both mutant and wild type siRNAs (SEQ ID NO:6,10) did not trigger effective cleavage of either the perfectly matched or mismatched target RNA (Fig. 1A). Thus, these siRNA sequences are inherently poor triggers of RNAi. On the other hand, P9 (SEQ ID NO: 14) and P10 wild type (SEQ ID NO:12) siRNAs triggered rapid cleavage of their corresponding the wild type target, but also produced significant cleavage of the mutant RNA (Fig. 1A). These siRNAs are good triggers of RNAi, but show poor selectivity. P10 mutant siRNA (SEQ ID NO:4) showed efficient and robust discrimination between mutant and wild type SOD1 RNAs (SEQ ID NO:7,8), cleaving the mutant RNA far more efficiently than the wild type (Fig. 1A). Most importantly, P10 mutant siRNA (SEQ ID NO:4) showed virtually complete discrimination between mutant and wild type SOD1 mRNA targets (Fig. 1A). This P10 mutant siRNA mediated efficient cleavage of the mutant SOD1 target but nearly no cleavage of the wild-type SOD1 mRNA (Fig. 1B), suggesting that this siRNA is ideal for therapeutic application.

### EXAMPLE III: Selective inhibition of mutant SOD1 G85R expression in Hela cells

To test whether cell-free reactions accurately predict siRNA efficiently and selectivity in mammalian cells, plasmid constructs that expressed the wild type or the mutant SOD1 G85R with GFP fused to their carboxyl termini were made. Each construct was transfected into Hela cells with a dsRed-expressing vector as a transfection control. The expression of either mutant or wild-type SOD1 (SEQ ID NO:7,8) was monitored by fluorescence-activated cell sorting (FACS) quantification of the green and red cells. Transfection of P9, P10 and P11 siRNAs with their corresponding mutant or wild type targets suppressed gene expression, although with different efficiencies and selectivites (Fig. 2). In contrast, co-transfection with a siRNA complementary to firefly luciferase did not suppress either the mutant or the wild type SOD1 expression (Fig. 2). All siRNAs did not suppress the mRNA targets with a single nucleotide mismatch except the siRNA p10 against wild type, which suppressed both the wild type and the mutant SOD1 expression effectively (Fig. 2). This result in general agrees with the *in-vitro* data (Fig. 1) and indicated that some, but not all siRNAs can efficiently discriminate the mRNA targets with a single-nucleotide difference.

### Example IV: Selective inhibition of mutant SOD1 G93A expression by U6-G93A vector in Hela cells

Recently it has been shown that shRNA can trigger RNAi *in-vivo.* To test whether shRNA against mutant SOD1 can selectively block the expression of the mutant but not the wild-type SOD1 expression, a plasmid was constructed that synthesized an shRNA homologous to another disease-causing mutant SOD1 G93A (nucleotide change from G to C at nucleotide position 281; placing a G:G mismatch at selective sites between the shRNA and wild-type SOD1; SEQ ID NO:16) under the control of a RNA polymerase III (U6) promoter (Sui et al., 2002). Results showed that when co-transfected with either wild-type or mutant SOD 1-GFP plasmids, this construct can be used to trigger single-nucleotide selective RNAi of mutant SOD1 in cultured cells (Fig. 3).

### EXAMPLE V: Selective inhibition of mutant SOD1 expression by siRNA and U6-G93A vector in-vivo

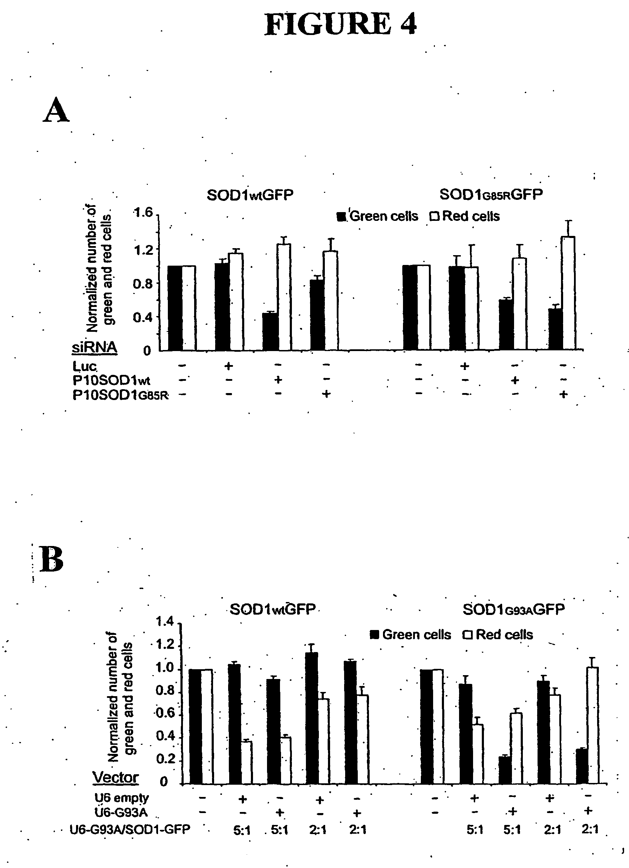

To test whether mutant selective inhibition can be achieved in neuronal cells, wild-type and mutant SOD1-GFP constructs were co-transfected the with either siRNA P10 against SOD1 G85R or shRNA-synthesizing vector against SOD G93A (SEQ ID NO:16) into a neuroblastoma cell line N2a. Similar to Hela cells, both synthetic siRNA and shRNA constructs directed the selective inhibition of mutant SOD1 expression in N2a cells (Fig. 4A, B).

### EXAMPLE VI: Selective inhibition of mutant SOD1 G85R in-vivo

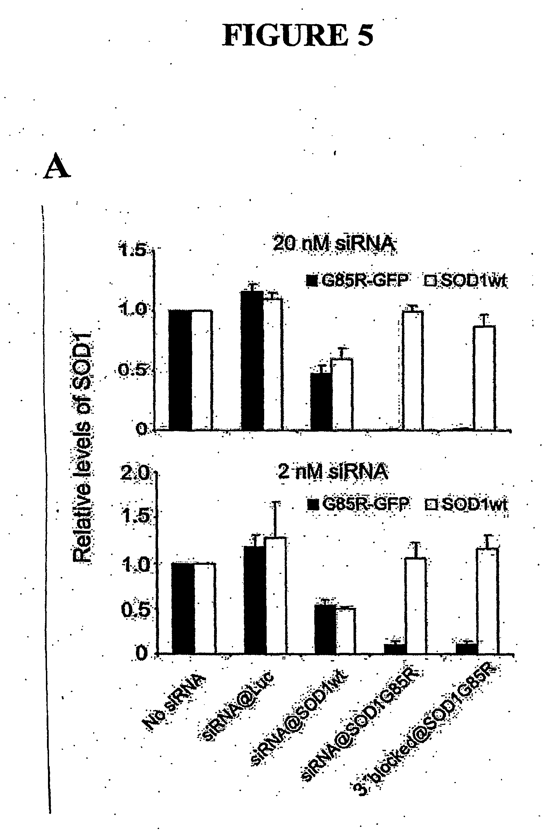

To determine whether single-nucleotide selective siRNA can discriminate between the mutant and the wild-type SOD1 when both mRNA's are present in the same cell, Hela cells were transfected with P10 siRNAs and mutant SOD1 G85R-GFP. Immunoblotting with anti-SOD1 antibodies were performed, which allowed for the detection of both the transfected fusion SOD1-GFP and the endogenous wild type human SOD1. The near 50% inhibition of the endogenous wild-type SOD1 expression reflected the transfection efficiency, which was ∼50%. In contrast to the P10 wild-type siRNA, at two different doses, P10 siRNA against the mutant inhibited expression of the mutant, but had no effect on the expression of endogenous wild-type SOD1 (Fig. 5).

### EXAMPLE VII: Selective inhibition of mutant SOD1 expression by U6-G93A vector in-vivo

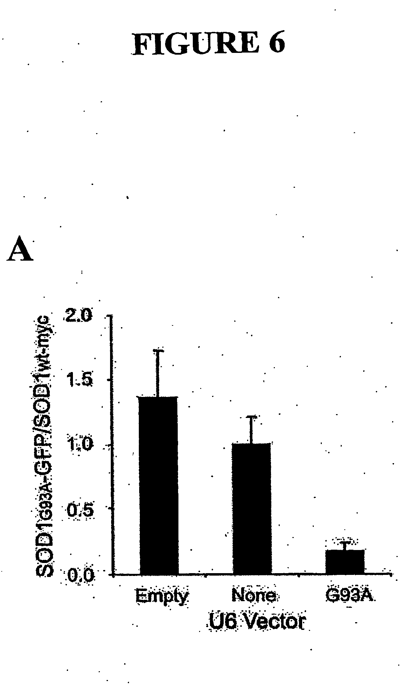

To test whether selective inhibition can occur *in-vivo,* transfection of SOD 1 reporters and shRNA plasmid into mice using a hydrodynamic transfection protocol was performed. The mutant SOD1 G93A-GFP plasmid and a wild type human SOD1 tagged with myc (which allowed better separation of the transfected human SOD1 from the endogenous mouse SOD1 on gels) were co-transfected with either U6 empty vector or U6-G93A vector. Liver expression of SOD1 G93A-GFP and SOD1myc was examined by Western blot. Results showed that only co-transfection with U6-G93A selectively decreased G93A expression (Fig. 6).

### EXAMPLE VIII: shRNA suppression of mutant SOD1 in-vivo using transgenic mice

To determine whether shRNA against mutant SOD1 can suppress mutant SOD1 expression in vivo, transgenic mice expressing shRNAs against SOD1^{G93A} under the control of a RNA polymerase III (Pol III) promoter U6 (U6-G93A mice) were made in a C57BL/6J and SJL hybrid background.

The plasmid synthesizing shRNA homologous mutant SOD1^{G93A} (shG93A) under the control of mouse U6 promoter was made according to the published protocol (Sui et al., 2002 Proc Natl Acad Sci USA 99:5515-5520). To make the mice, the transgene was linearized by digestion using Kpn I and Sac I, purified and injected into fertilized mouse eggs at University of Massachusetts Medical School (UMMS) transgenic core.

To screen for U6-G93A transgenic mice, PCR primers that selectively amplify the transgene sequence were designed and used to identify the transgenic mice. A total of seven founders (F0) were identified. These founders have been crossed with mice transgenic for mutant SOD1^{G93A} in an FVB background. F1 mice were analyzed for transgene copy numbers using Southern blot as described previously (Xu et al., 1993 Cell 73:23-33). Tail DNA was digested with Bam H1, which generated a transgene fragment of 388 nucleotides. Because the endogenous mouse U6 promoter has only one BamHI site, the BamHI digestion produced a larger fragment from the endogenous mouse U6 gene. ³²P-labeled RNA oligonucleotide probes complementary to the U6 promoter region were used for hybridization. The U6 region was used as the target because the endogenous mouse U6 band can be detected together with the transgene on the same blot, therefore, the endogenous band can be used as the reference for quantifying the transgene copy number.

The U6-G93A shRNA construct was expressed in cells from the double transgenic mice as measured using Northern blots. The U6-G93A shRNA construct was found to silence expression of mutant SOD1^{G93A} in the double transgenic mice (expressing the U6-G93A shRNA construct and mutant SOD1^{G93A}) as measured using Western blots

### Discussion of Results Examples I-VIII

The possibility of using RNAi to selectively silence a dominant mutant ALS gene was investigated. Using multiple siRNAs matching either wild-type or mutant SOD1, results showed that siRNAs against mutant SOD1 G85R cleave the mutant, but not the wild-type SOD1 RNA efficiently *in-vitro* (Fig. 1). In addition, these siRNAs selectively inhibited the mutant but not the wild-type SOD1 protein expression in mammalian cell (Fig. 2), even when both the mutant and the wild type proteins were present in the same cells (Fig. 4). A vector expressing a hairpin that is processed *in-vivo* into an siRNA also selectively inhibited mutant but not wild-type SOD1 expression in mouse liver (Figs. 3, 4, 6). These results demonstrated that selective inhibition of a dominant mutant allele can be achieved using RNAi and optimal siRNA and shRNA sequences can be identified by a pre-clinical screen *in-vitro* or *in-vivo.*

Although SOD1 single nucleotide discrimination can be achieved in mammalian cells, this discrimination is not guaranteed. Some siRNAs are capable of discrimination between alleles that differ at a single nucleotide while others cannot. Results point to two different types of deficiencies for siRNA designed to target mutant, disease causing alleles. First, while siRNAs perfectly matched to their target can cleave their target and inhibit the protein expression from the target gene, all siRNAs do not silence with the same efficiency. For example, among the siRNAs against the wild type, P9 and P10 cleaved their target more efficiently than P11 *in-vitro* (Fig. 1). P10 also inhibited target gene expression most efficiently in mammalian cells (Fig. 2). Similarly, among the siRNAs against the mutant SOD1 G85R, P9 and P10 cleaved the mutant RNA more efficiently than P11 (Fig. 1). P10 was also the most efficient in inhibiting the mutant SOD1 expression in mammalian cells (Fig. 2). It is intriguing that a single nucleotide shift of the siRNA sequence against the target results in such a significant change in silencing efficiency. Second, differences in selectivity between the perfectly matched target RNA and the RNA bearing a single nucleotide mismatch were observed among six siRNAs used. For example, wild- type P10 siRNA conferred poor selectivity. Wild-type P10 cleaved both wild type and mutant SOD1 RNA in the cell-free assay and inhibited the expression of both alleles in mammalian cells with high efficiency (Figs 1, 2, 4, 5). On the other hand, P10 siRNA directed against mutant SOD1 conferred the highest selectivity. It cleaved the mutant SOD1 RNA and inhibited the mutant SOD1 expression in cell-free assay and inhibited mutant but not wild-type SOD1 expression in mammalian cells (Figs. 1-6).

An explanation for the different selectivity between the mutant and the wild type P10 siRNAs is the following: the mismatch between the mutant P10 siRNA and the wild type SOD1 mRNA created a G:G clash, while the mismatch between the wild type P10 siRNA and the mutant G85R mRNA resulted in a C:C clash (see Fig. 1A). Thus, in designing an siRNA that selectively acts on one allele of a given sequence, the following are considered. Without wishing to be bound by theory, a purine:purine mismatch disrupts the A-form helix that is required between the antisense strand of the siRNA and its mRNA target (Chiu et al., 2002). In contrast, a pyrimidine:pyrimidine mismatch may more readily be accommodated within an A-form helix. Thus, the G:G clash between the siRNA and the wild-type target RNA discriminates against the wild-type target, producing greater selectivity for the mutant target. Noticeably, the siRNA hairpin vector against G93A, which showed a good selectivity for mutant SOD1, also created a G:G clash with the wild-type SOD1 mRNA. These results suggested that purine:purine mismatches confer greater selectivity than pyrimidine:pyrimidine mismatches. In addition to designing siRNAs for use in the present method that contain pyrimidine:pyrimidine mismatches, the siRNAs are designed using methods known in the art.

### REFERENCES

1. Amarzguioui, M., Holen, T., Babaie, E., and Prydz, H. (2003). Tolerance for mutations and chemical modifications in a siRNA. Nucleic Acids Res 31, 589-595.
2. Behndig, A., Karlsson, K., Reaume, A. G., Sentman, M. L. & Marklund, S. L. In vitro photochemical cataract in mice lacking copper-zinc superoxide dismutase. Free Radic Biol Med 31, 738-44. (2001).
3. Boutla, A., Delidakis, C., Livadaras, I., Tsagris, M. & Tabler, M. Short 5'-phosphorylated double-stranded RNAs induce RNA interference in Drosophila. Curr Biol 11, 1776-80. (2001).
4. Brummelkamp, T. R., Bernards, R. & Agami, R. A system for stable expression of short interfering RNAs in mammalian cells. Science 296, 550-3. (2002).
5. Brummelkamp, T., Bernards, R. & Agami, R. Stable suppression of tumorigenicity by virus-mediated RNA interference. Cancer Cell 2, 243. (2002).
6. Caplen, N. J., Parrish, S., Imani, F., Fire, A. & Morgan, R. A. Specific inhibition of gene expression by small double-stranded RNAs in invertebrate and vertebrate systems. Proc Natl Acad Sci U S A 98, 9742-7. (2001).
7. Caplen, N. J. et al. Rescue of polyglutamine-mediated cytotoxicity by double-stranded RNA-mediated RNA interference. Hum Mol Genet 11, 175-84. (2002).
8. Chiu, Y.-L. & Rana, T. M. RNAi in human cells: basic structural and functional features of small interfering RNA. Molecular Cell 10, 549-561 (2002).
9. Cleveland, D. W. & Rothstein, J. D. From Charcot to Lou Gehrig: deciphering selective motor neuron death in ALS. Nat Rev Neurosci 2, 806-19. (2001).
10. Devroe, E. & Silver, P. A. Retrovirus-delivered siRNA. BMC Biotechnol 2, 15. (2002).
11. Elbashir, S. M., Lendeckel, W. & Tuschl, T. RNA interference is mediated by 21- and 22-nucleotide RNAs. Genes Dev 15, 188-200. (2001).
12. Elbashir, S. M. et al. Duplexes of 21-nucleotide RNAs mediate RNA interference in cultured mammalian cells. Nature 411, 494-8. (2001).
13. Elbashir, S. M., Martinez, J., Patkaniowska, A., Lendeckel, W. & Tuschl, T. Functional anatomy of siRNAs for mediating efficient RNAi in Drosophila melanogaster embryo lysate. Embo J 20, 6877-88. (2001).
14. Flood, D. G. et al. Hindlimb motor neurons require Cu/Zn superoxide dismutase for maintenance of neuromuscular junctions. Am J Pathol 155, 663-72. (1999).
15. Hamilton, A. J. & Baulcombe, D. C. A species of small antisense RNA in posttranscriptional gene silencing in plants. Science 286, 950-2. (1999).
16. Hammond, S. M., Bernstein, E., Beach, D. & Hannon, G. J. An RNA-directed nuclease mediates post-transcriptional gene silencing in Drosophila cells. Nature 404, 293-6. (2000).
17. Hannon, G. J. RNA interference. Nature 418, 244-51. (2002).
18. Hutvagner, G. & Zamore, P. D. RNAi: nature abhors a double-strand. Curr Opin Genet Dev 12, 225-32. (2002).
19. Jacque, J. M., Triques, K. & Stevenson, M. Modulation of EW-1 replication by RNA interference. Nature 418, 435-8. (2002).
20. Kawase, M. et al. Exacerbation of delayed cell injury after transient global ischemia in mutant mice with CuZn superoxide dismutase deficiency. Stroke 30, 1962-8. (1999).
21. Kondo, T. et al. Reduction of CuZn-Superoxide Dismutase Activity Exacerbates Neuronal Cell Injury and Edema Formation after Transient Focal Cerebral Ischemia. Journal ofNeuroscience 17, 4180-9 (1997).
22. Lee, N. S. et al. Expression of small interfering RNAs targeted against IHV-1 rev transcripts in human cells. Nat Biotechnol 20, 500-5. (2002).
23. Lipardi, C., Wei, Q. & Paterson, B. M. RNAi as random degradative PCR: siRNA primers convert mRNA into dsRNAs that are degraded to generate new siRNAs. Cell 107, 297-307. (2001).
24. Matz, P. G., Copin, J. C. & Chan, P. H. Cell death after exposure to subarachnoid hemolysate correlates inversely with expression of CuZn-superoxide dismutase. Stroke 31, 2450-9. (2000).
25. Matzuk, M. M., Dionne, L., Guo, Q., Kumar, T. R. & Lebovitz, R. M. Ovarian function in superoxide dismutase 1 and 2 knockout mice. Endocrinology 139, 4008-11. (1998).
26. McFadden, S. L., Ding, D. & Salvi, R. Anatomical, metabolic and genetic aspects of age-related hearing loss in mice. Audiology 40, 313-21. (2001).
27. McManus, M. T. & Sharp, P. A. Gene silencing in mammals by small interfering RNAs. Nat Rev Genet 3, 737-47. (2002).
28. McManus, M. T., Petersen, C. P., Haines, B. B., Chen, J. & Sharp, P. A. Gene silencing using micro-RNA designed hairpins. Rna 8, 842-50. (2002).
29. Miyagishi, M. & Taira, K. U6 promoter-driven siRNAs with four uridine 3' overhangs efficiently suppress targeted gene expression in mammalian cells. Nat Biotechnol 20, 497-500. (2002).
30. Nykanen, A., Haley, B. & Zamore, P. D. ATP requirements and small interfering RNA structure in the RNA interference pathway. Cell 107, 309-21. (2001).
31. Paddison, P. J., Caudy, A. A., Bernstein, E., Hannon, G. J. & Conklin, D. S. Short hairpin RNAs (shRNAs) induce sequence-specific silencing in mammalian cells. Genes Dev 16,948-58. (2002).
32. Paul, C. P., Good, P. D., Winer, I. & Engelke, D. R. Effective expression of small interfering RNA in human cells. Nat Biotechnol 20, 505-8. (2002).
33. Shefner, J. M. et al. Mice lacking cytosolic copper/zinc superoxide dismutase display a distinctive motor axonopathy. Neurology 53, 1239-46. (1999).
34. Schwarz, D. S., Hutvagner, G., Haley, B. & Zamore, P. D. Evidence that siRNAs function as guides, not primers, in the Drosophila and human RNAi pathways. Molecular Cell 10, 537-548 (2002).
35. Sui, G. et al. A DNA vector-based RNAi technology to suppress gene expression in mammalian cells. Proc Natl Acad Sci U S A 99, 5515-20. (2002).
36. Taylor, J. P., Hardy, J. & Fischbeck, K. H. Toxic proteins in neurodegenerative disease. Science 296, 1991-5. (2002).
37. Tuschl, T., Zamore, P. D., Lehmann, R., Bartel, D. P. & Sharp, P. A. Targeted mRNA degradation by double-stranded RNA in vitro. Genes Dev 13, 3191-7. (1999).
38. Xia, H., Mao, Q., Paulson, H. L. & Davidson, B. L. siRNA-mediated gene silencing in vitro and in vivo. Nat Biotechnol 20, 1006-10. (2002).
39. Xu, P., Vernooy, S. Y., Guo, M., and Hay, B. A. (2003). The Drosophila MicroRNA Mir-14 Suppresses Cell Death and Is Required for Normal Fat Metabolism. Curr Biol 13, 790-795.
40. Yu, J. Y., DeRuiter, S. L. & Turner, D. L. RNA interference by expression of short-interfering RNAs and hairpin RNAs in mammalian cells. Proc Natl Acad Sci U S A 99, 6047-52. (2002).
41. Zamore, P. D., Tuschl, T., Sharp, P.A. & Bartel, D. P. RNAi: double-stranded RNA directs the ATP-dependent cleavage of mRNA at 21 to 23 nucleotide intervals. Cell 101, 25-33. (2000).
42. Zeng, Y., Wagner, E. J. & Cullen, B. R. Both natural and designed micro RNAs can inhibit the expression of cognate mRNAs when expressed in human cells. Mol Cell 9, 1327-33. (2002).

## Claims

1. An *ex vivo* method of selectively inhibiting expression of a mutant target allele of a SOD1 gene in a cell comprising wild-type and mutant alleles of the gene, wherein the target allele comprises a point mutation correlated with amyotrophic lateral sclerosis, the method comprising administering to the cell an siRNA specific for the point mutation such that allele-specific RNA interference of the mutant target allele occurs and expression of the wild-type allele is preserved, wherein the siRNA is matched completely with a mutant mRNA encoded by the mutant allele but forms a single nucleotide mismatch with a wild-type mRNA encoded by the wild-type allele.

2. The method of claim 1, wherein the single nucleotide mismatch is a purine: purine mismatch.

3. The method of claim 2, wherein the mismatch is a G:G mismatch.

4. The method of claim 1, wherein the point mutation is a dominant gain of function mutation.

5. The method of claims 1, where the mutant and wild-type alleles differ by:
(a) only one, two, or three nucleotides; or
(b) a single nucleotide.

6. The method of any of claims 2 to 5, wherein the mismatch is located at nucleotide position 9 (P9) relative to the 5' end of the antisense strand of the siRNA, or position 10 (P10) relative to the 5' end of the antisense strand of the siRNA.

7. The method of claim 1, wherein the mutant allele encodes a glycine to arginine mutation at amino acid position 85 (G85) of a mutant SOD1 protein, or a glycine to alanine mutation at amino acid position 93 (G93A) of a mutant SOD1 protein.

8. The method of claim 1, wherein the siRNA is either:
(a) a siRNA comprising (i) a sense strand sequence corresponding to the sequence set forth as SEQ ID NO:3; and (ii) an antisense-strand sequence set forth as SEQ ID NO:4; or
(b) a siRNA comprising (i) a sense strand sequence corresponding to the sequence set forth as SEQ ID NO:1; and (ii) an antisense-strand sequence set forth as SEQ ID NO:2.

9. The method of claim 1, wherein the siRNA is administered to the cell in the form of a shRNA, wherein the shRNA is cleaved in the cell to generate the siRNA.

10. The method of claim 9, wherein shRNA is a G93A SOD1 shRNA.

11. The method of claim 10, wherein the shRNA has the sequence set forth as SEQ ID NO:16.

12. The method of claim 9, wherein the shRNA is expressed from an expression construct.

13. The method of claim 12, wherein the shRNA is expressed under the control of a RNA polymerase III (U6) promoter.

14. An siRNA for treating amyotrophic lateral sclerosis associated with a mutant protein encoded by a mutant target allele of a SOD1 gene in a subject, wherein the siRNA selectively inhibits expression of the mutant target allele, wherein the subject has wild-type and mutant alleles of the gene, wherein the siRNA is specific for a point mutation in the mutant target allele, such that allele-specific RNA interference of the mutant target allele occurs and expression of the wild-type allele is preserved, wherein the siRNA is matched completely with a mutant mRNA encoded by the mutant allele but comprises a single nucleotide mismatch with a wild-type mRNA encoded by the wild-type allele.

15. The siRNA of claim 14, wherein the single nucleotide mismatch is a purine:purine mismatch.

16. The siRNA of claim 15, wherein the mismatch is a G:G mismatch.

17. The siRNA of claim 14, wherein the point mutation is a dominant gain of function mutation.

18. The siRNA of claim 14, wherein the mutant and wild-type alleles differ by:
(a) only one, two, or three nucleotides; or
(b) a single nucleotide.

19. The siRNA of claim 14, wherein the mismatch is located at either nucleotide position 9 (P9) relative to the 5' end of the antisense strand of the siRNA, or nucleotide position 10 (P10) relative to the 5' end of the antisense strand of the siRNA.

20. The siRNA of claim 14, wherein the mutant allele encodes a glycine to arginine mutation at amino acid position 85 (G85R) of a mutant SOD1 protein, or a glycine to alanine mutation at amino acid position 93 (G93A) of a mutant SOD1 protein.

21. The siRNA of claim 20, wherein the siRNA is
(a) a siRNA comprising (i) a sense strand sequence corresponding to the sequence set forth as SEQ ID NO:3; and (ii) an antisense-strand sequence set forth as SEQ ID NO:4; or
(b) a siRNA comprising (i) a sense strand sequence corresponding to the sequence set forth as SEQ ID NO:1; and (ii) an antisense-strand sequence set forth as SEQ ID NO:2.

22. The siRNA of claim 14, wherein the siRNA is administered to the cell in the form of a shRNA, wherein the shRNA is cleaved in the cell to generate the siRNA.

23. The siRNA of claim 22, wherein shRNA is a G93A SOD1 shRNA

24. The siRNA of claim 23, wherein the shRNA has the sequence set forth as SEQ ID NO:16.

25. The siRNA of claim 22, wherein the shRNA is expressed from an expression construct.

26. The siRNA of claim 25, wherein the shRNA is expressed under the control of a RNA polymerase III (U6) promoter.

## Patentansprüche

1. Ein *ex vivo* Verfahren zur selektiven Hemmung der Expression eines mutierten Zielallels eines SOD1-Gens in einer Zelle, umfassend Wildtyp- und mutiertes Allel des Gens, wobei das Zielallel eine mit amyotropher Lateralsklerose korrelierte Punktmutation umfasst, wobei das Verfahren die Verabreichung der Zelle einer siRNA umfasst, die für die Punktmutation spezifisch ist, so dass eine allelspezifische RNA-Interferenz des mutierten Ziellallels erfolgt und eine Expression des Wildtyp-Allels aufrechterhalten wird, wobei die siRNA vollständig mit einer mutierten mRNA gepaart ist, die von dem mutierten Allel codiert ist, aber eine Einzelnukleotid-Fehlpaarung mit einer Wildtyp-mRNA bildet, die von dem Wildtyp-Allel codiert ist.

2. Das Verfahren nach Anspruch 1, wobei die Einzelnukleotid-Fehlpaarung eine Purin:Purin-Fehlpaarung ist.

3. Das Verfahren nach Anspruch 2, wobei die Fehlpaarung eine G:G-Fehlpaarung ist.

4. Das Verfahren nach Anspruch 1, wobei die Punktmutation eine dominante gain-of function Mutation ist.

5. Das Verfahren nach Anspruch 1, wobei das mutierte Allel und Wildtyp-Allel sich unterscheiden durch:
(a) nur ein, zwei oder drei Nukleotide; oder
(b) ein Einzelnukleotid.

6. Das Verfahren nach einem der Ansprüche 2 bis 5, wobei die Fehlpaarung an Nukleotidposition 9 (P9) bezogen auf das 5'-Ende des Antisense-Strangs der siRNA oder an Position 10 (P10) bezogen auf das 5'-Ende des Antisense-Strangs der siRNA lokalisiert ist.

7. Das Verfahren nach Anspruch 1, wobei das mutierte Allel eine Mutation von Glycin zu Arginin an Aminosäureposition 85 (G85R) eines mutierten SOD1-Proteins oder eine Mutation von Glycin zu Alanin an Aminosäureposition 93 (G93A) eines mutierten SOD1-Proteins codiert.

8. Das Verfahren nach Anspruch 1, wobei die siRNA entweder:
(a) eine siRNA ist, umfassend (i) eine Sensestrang-Sequenz, die der als SEQ ID NR:3 gezeigten Sequenz entspricht; und (ii) eine Antisensestrang-Sequenz, die als SEQ ID NR:4 gezeigt ist; oder
(b) eine siRNA ist, umfassend (i) eine Sensestrang-Sequenz, die der als SEQ ID NR:1 gezeigten Sequenz entspricht; und (ii) eine Antisensestrang-Sequenz, die als SEQ ID NR:2 gezeigt ist.

9. Das Verfahren nach Anspruch 1, wobei die siRNA in Form einer shRNA der Zelle verabreicht wird, wobei die shRNA in der Zelle geschnitten wird, um die siRNA zu bilden.

10. Das Verfahren nach Anspruch 9, wobei die shRNA eine G93A SOD1 shRNA ist.

11. Das Verfahren nach Anspruch 10, wobei die shRNA die als SEQ ID NR:16 gezeigte Sequenz besitzt.

12. Das Verfahren nach Anspruch 9, wobei die shRNA von einem Expressionskonstrukt exprimiert wird.

13. Das Verfahren nach Anspruch 12, wobei die shRNA unter der Kontrolle eines RNA-Polymerase III (U6)-Promotors exprimiert wird.

14. Eine siRNA zur Behandlung der amyotrophen Lateralsklerose, die mit einem mutierten Protein verbunden ist, das von einem mutierten Zielallel eines SOD1-Gens codiert ist, in einem Subjekt, wobei die siRNA die Expression des mutierten Zielallels selektiv hemmt, wobei das Subjekt Wildtyp- und mutiertes Allel des Gens besitzt, wobei die siRNA für eine Punktmutation in dem mutierten Zielallel spezifisch ist, so dass eine allelspezifische RNA-Interferenz des mutierten Ziellallels erfolgt und eine Expression des Wildtyp-Allels aufrechterhalten wird, wobei die siRNA vollständig mit einer mutierten mRNA gepaart ist, die von dem mutierten Allel codiert ist, aber eine Einzelnukleotid-Fehlpaarung mit einer Wildtyp-mRNA umfasst, die von dem Wildtyp-Allel codiert ist.

15. Die siRNA nach Anspruch 14, wobei die Einzelnukleotid-Fehlpaarung eine Purin:Purin-Fehlpaarung ist.

16. Die siRNA nach Anspruch 15, wobei die Fehlpaarung eine G:G-Fehlpaarung ist.

17. Die siRNA nach Anspruch 14, wobei die Punktmutation eine dominante gain-of function Mutation ist.

18. Die siRNA nach Anspruch 14, wobei das mutierte Allel und Wildtyp-Allel sich unterscheiden durch:
(a) nur ein, zwei oder drei Nukleotide; oder
(b) ein Einzelnukleotid.

19. Die siRNA nach Anspruch 14, wobei die Fehlpaarung entweder an Nukleotidposition 9 (P9) bezogen auf das 5'-Ende des Antisense-Strangs der siRNA oder an Nukleotidposition 10 (P10) bezogen auf das 5'-Ende des Antisense-Strangs der siRNA lokalisiert ist.

20. Die siRNA nach Anspruch 14, wobei das mutierte Allel eine Mutation von Glycin zu Arginin an Aminosäureposition 85 (G85R) eines mutierten SOD1-Proteins oder eine Mutation von Glycin zu Alanin an Aminosäureposition 93 (G93A) eines mutierten SOD1-Proteins codiert.

21. Die siRNA nach Anspruch 20, wobei die siRNA:
(a) eine siRNA ist, umfassend (i) eine Sensestrang-Sequenz, die der als SEQ ID NR:3 gezeigten Sequenz entspricht; und (ii) eine Antisensestrang-Sequenz, die als SEQ ID NR:4 gezeigt ist; oder
(b) eine siRNA ist, umfassend (i) eine Sensestrang-Sequenz, die der als SEQ ID NR:1 gezeigten Sequenz entspricht; und (ii) eine Antisensestrang-Sequenz, die als SEQ ID NR:2 gezeigt ist.

22. Die siRNA nach Anspruch 14, wobei die siRNA in Form einer shRNA der Zelle verabreicht wird, wobei die shRNA in der Zelle geschnitten wird, um die siRNA zu bilden.

23. Die siRNA nach Anspruch 22, wobei die shRNA eine G93A SOD1 shRNA ist.

24. Die siRNA nach Anspruch 23, wobei die shRNA die als SEQ ID NR:16 gezeigte Sequenz besitzt.

25. Die siRNA nach Anspruch 22, wobei die shRNA von einem Expressionskonstrukt exprimiert wird.

26. Die siRNA nach Anspruch 25, wobei die shRNA unter der Kontrolle eines RNA-Polymerase III (U6)-Promotors exprimiert wird.

## Revendications

1. Procédé *ex vivo* d'inhibition sélective de l'expression d'un allèle cible mutant d'un gène SOD1 dans une cellule comprenant des allèles mutants et de type sauvage du gène, ledit allèle cible comprenant une mutation ponctuelle qui est corrélée avec la sclérose latérale amyotrophique, le procédé comprenant l'administration à la cellule d'un ARNsi spécifique à la mutation ponctuelle de façon à produire une interférence d'ARN allèle-spécifique de l'allèle cible mutant et à préserver l'expression de l'allèle de type sauvage, l'ARNsi étant complètement apparié avec un ARNm mutant codé par l'allèle mutant mais formant un mésappariement nucléotidique simple avec un ARNm de type sauvage codé par l'allèle de type sauvage.

2. Procédé selon la revendication 1, dans lequel le mésappariement nucléotidique simple est un mésappariement purine:purine.

3. Procédé selon la revendication 2, dans lequel le mésappariement est un mésappariement G:G.

4. Procédé selon la revendication 1, dans lequel la mutation ponctuelle est une mutation dominante par gain de fonction.

5. Procédé selon la revendication 1, dans lequel les allèles mutants et de type sauvage diffèrent :
(a) d'un seul, de deux ou de trois nucléotides ; ou
(b) d'un nucléotide simple.

6. Procédé selon l'une quelconque des revendications 2 à 5, dans lequel le mésappariement se situe à la position nucléotidique 9 (P9) par rapport à l'extrémité 5' du brin antisens de l'ARNsi ou à la position 10 (P10) par rapport à l'extrémité 5' du brin antisens de l'ARNsi.

7. Procédé selon la revendication 1, dans lequel l'allèle mutant code pour une mutation de glycine en arginine à la position d'acide aminé 85 (G85R) d'une protéine SOD1 mutante ou pour une mutation de glycine en alanine à la position d'acide aminé 93 (G93A) d'une protéine SOD1 mutante.

8. Procédé selon la revendication 1, dans lequel l'ARNsi est :
(a) soit un ARNsi comprenant (i) une séquence de brin sens correspondant à la séquence référencée SEQ ID N°3 ; et (ii) une séquence de brin antisens référencée SEQ ID N°4 ;
(b) soit un ARNsi comprenant (i) une séquence de brin sens correspondant à la séquence référencée SEQ ID N°1; et (ii) une séquence de brin antisens référencée SEQ ID N°2.

9. Procédé selon la revendication 1, dans lequel ARNsi est administré à la cellule sous la forme d'un ARNsh, ledit ARNsh étant coupé dans la cellule afin de générer ARNsi.

10. Procédé selon la revendication 9, dans lequel l'ARNsh est un ARNsh de SOD1 G93A.

11. Procédé selon la revendication 10, dans lequel l'ARNsh a la séquence référencée SEQ ID N°16.

12. Procédé selon la revendication 9, dans lequel l'ARNsh est exprimé à partir d'un produit d'assemblage d'expression.

13. Procédé selon la revendication 12, dans lequel l'ARNsh est exprimé sous le contrôle d'un promoteur de l'ARN polymérase III (U6).

14. ARNsi destiné au traitement de la sclérose latérale amyotrophique associée à une protéine mutante codée par un allèle cible mutant d'un gène SOD1 chez un sujet, l'ARNsi inhibant de manière sélective l'expression de l'allèle cible mutant, le sujet possédant des allèles de type sauvage et mutants du gène, l'ARNsi étant spécifique à une mutation ponctuelle dans l'allèle cible mutant permettant de produire une interférence d'ARN allèle-spécifique de l'allèle cible mutant et de préserver l'expression de l'allèle de type sauvage, l'ARNsi étant complètement apparié avec un ARNm mutant codé par l'allèle mutant mais comprenant un mésappariement nucléotidique simple avec un ARNm de type sauvage codé par l'allèle de type sauvage.

15. ARNsi selon la revendication 14, dans lequel le mésappariement nucléotidique simple est un mésappariement purine:purine.

16. ARNsi selon la revendication 15, dans lequel le mésappariement est un mésappariement G:G.

17. ARNsi selon la revendication 14, dans lequel la mutation ponctuelle est une mutation dominante par gain de fonction.

18. ARNsi selon la revendication 14, dans lequel les allèles mutants et de type sauvage diffèrent :
(a) d'un seul, de deux ou de trois nucléotides ; ou
(b) d'un nucléotide simple.

19. ARNsi selon la revendication 14, dans lequel le mésappariement se situe soit à la position nucléotidique 9 (P9) par rapport à l'extrémité 5' du brin antisens de l'ARNsi, soit à la position nucléotidique 10 (P10) par rapport à l'extrémité 5' du brin antisens de ARNsi.

20. ARNsi selon la revendication 14, dans lequel l'allèle mutant code pour une mutation de glycine en arginine à la position d'acide aminé 85 (G85R) d'une protéine SOD1 mutante ou pour une mutation de glycine en alanine à la position d'acide aminé 93 (G93A) d'une protéine SOD1 mutante.

21. ARNsi selon la revendication 20, dans lequel l'ARNsi est
(a) soit un ARNsi comprenant (i) une séquence de brin sens correspondant à la séquence référencée SEQ ID N°3 ; et (ii) une séquence de brin antisens référencée SEQ ID N°4 ;
(b) soit un ARNsi comprenant (i) une séquence de brin sens correspondant à la séquence référencée SEQ ID N°1; et (ii) une séquence de brin antisens référencée SEQ ID N°2.

22. ARNsi selon la revendication 14, dans lequel l'ARNsi est administré à la cellule sous la forme d'un ARNsh, ledit ARNsh étant coupé dans la cellule afin de générer l'ARNsi.

23. ARNsi selon la revendication 22, dans lequel l'ARNsh est un ARNsh de SOD1 G93A.

24. ARNsi selon la revendication 23, dans lequel l'ARNsh a la séquence référencée SEQ ID N°16.

25. ARNsi selon la revendication 22, dans lequel l'ARNsh est exprimé à partir d'un produit d'assemblage d'expression.

26. ARNsi selon la revendication 25, dans lequel l'ARNsh est exprimé sous le contrôle d'un promoteur de l'ARN polymérase III (U6).


### SEQUENCE LISTING

<110> University of Massachusetts et al.
<120> Allele-Specific RNA Interference
<130> UMY-038PC
<150> 60/423,507
   <151> 2002-11-04
<150> 60/488,283
   <151> 2003-07-18
<160> 19
<170> FastSEQ for Windows Version 4.0
<210> 1
   <211> 25
   <212> DNA
   <213> Homo sapiens
<400> 1
   uggagacuug cgcaaugugt ttttt 25
<210> 2
   <211> 25
   <212> DNA
   <213> Homo sapiens
<400> 2
   cacauugcgc aagucuccat ttttt 25
<210> 3
   <211> 25
   <212> DNA
   <213> Homo sapiens
<400> 3
   ggagacuugc gcaaugugat ttttt 25
<210> 4
   <211> 25
   <212> DNA
   <213> Homo sapiens
<400> 4
   ucacauugcg caagucucct ttttt 25
<210> 5
   <211> 25
   <212> DNA
   <213> Homo sapiens
<400> 5
   gagacuugcg caaugugact ttttt 25
<210> 6
   <211> 25
   <212> DNA
   <213> Homo sapiens
<400> 6
   gucacauugc gcaagucuct ttttt 25
<210> 7
   <211> 48
   <212> DNA
   <213> Homo sapiens
<400> 7
   gagaggcaug uuggagacuu gggcaaugug acugcugaca aagauggu 48
<210> 8
   <211> 48
   <212> DNA
   <213> Homo sapiens
<400> 8
   gagaggcaug uuggagacuu gcgcaaugug acugcugaca aagauggu 48
<210> 9
   <211> 25
   <212> DNA
   <213> Homo sapiens
<400> 9
   gagacuuggg caaugugact ttttt 25
<210> 10
   <211> 25 ,
   <212> DNA
   <213> Homo sapiens
<400> 10
   gucacauugc ccaagucuct ttttt 25
<210> 11
   <211> 25
   <212> DNA
   <213> Homo sapiens
<400> 11
   ggagacuugg gcaaugugat ttttt 25
<210> 12
   <211> 25
   <212> DNA
   <213> Homo sapiens
<400> 12
   ucacauugcc caagucucct ttttt 25
<210> 13
   <211> 25
   <212> DNA
   <213> Homo sapiens
<400> 13
   uggagacuug ggcaaugugt ttttt 25
<210> 14
   <211> 25
   <212> DNA
   <213> Homo sapiens
<400> 14
   cacauugccc aagucuccat ttttt 25
<210> 15
   <211> 35
   <212> DNA
   <213> Homo sapiens
<400> 15
   actgctgaca aagatggtgt ggccgatgtg tctat 35
<210> 16
   <211> 52
   <212> DNA
   <213> Homo sapiens
<400> 16
   gacaaagaug cuguggccga uaagcuuauc ggccacagca ucuuugucuu uu 52
<210> 17
   <211> 153
   <212> PRT
   <213> Homo sapiens
<400> 17
   Ala Thr Lys Ala Val Cys Val Leu Lys Gly Asp Gly Pro Val Gln Gly
   1               5                   10                  15
   Ile Ile Asn Phe Glu Gln Lys Glu Ser Asn Gly Pro Val Lys Val Trp
               20                  25                  30
   Gly Ser Ile Lys Gly Leu Thr Glu Gly Leu His Gly Phe His Val His
           35                  40                  45
   Glu Phe Gly Asp Asn Thr Ala Gly Cys Thr Ser Ala Gly Pro His Phe
       50                  55                  60
   Asn Pro Leu Ser Arg Lys His Gly Gly Pro Lys Asp Glu Glu Arg His
   65                  70                  75                  80
   Val Gly Asp Leu Gly Asn Val Thr Ala Asp Lys Asp Gly Val Ala Asp
                   85                  90                  95
   Val Ser Ile Glu Asp Ser Val Ile Ser Leu Ser Gly Asp His Cys Ile
               100                 105                 110
   Ile Gly Arg Thr Leu Val Val His Glu Lys Ala Asp Asp Leu Gly Lys
           115                 120                 125
   Gly Gly Asn Glu Glu Ser Thr Lys Thr Gly Asn Ala Gly Ser Arg Leu
       130                 135                 140
   Ala Cys Gly Val Ile Gly Ile Ala Gln
   145                 150
<210> 18
   <211> 459
   <212> DNA
   <213> Homo sapiens
<400> 18
   gcgacgaagg ccgtgtgcgt gctgaagggc gacggcccag tgcagggcat catcaatttc 60
   gagcagaagg aaagtaatgg accagtgaag gtgtggggaa gcattaaagg actgactgaa 120
   ggcctgcatg gattccatgt tcatgagttt ggagataata cagcaggctg taccagtgca 180
   ggtcctcact ttaatcctct atccagaaaa cacggtgggc caaaggatga agagaggcat 240
   gttggagact tgggcaatgt gactgctgac aaagatggtg tggccgatgt gtctattgaa 300
   gattctgtga tctcactctc aggagaccat tgcatcattg gccgcacact ggtggtccat 360
   gaaaaagcag atgacttggg caaaggtgga aatgaagaaa gtacaaagac aggaaacgct 420
   ggaagtcgtt tggcttgtgg tgtaattggg atcgcccaa                       459
<210> 19
   <211> 2288
   <212> DNA
   <213> Homo sapiens
<400> 19
   gtaccctgtt tacatcattt tgccattttc gcgtactgca accggcgggc cacgccgtga 60
   aaagaaggtt gttttctcca cagtttcggg gttctggacg tttcccggct gcggggcggg 120
   gggagtctcc ggcgcacgcg gccccttggc ccgccccagt cattcccggc cactcgcgac 180
   ccgaggctgc cgcagggggc gggctgagcg cgtgcgaggc cattggtttg gggccagagt 240
   gggcgaggcg cggaggtctg gcctataaag tagtcgcgga gacggggtgc tggtttgcgt 300
   cgtagtctcc tgcaggtctg gggtttccgt tgcagtcctc ggaaccagga cctcggcgtg 360
   gcctagcgag ttatggcgac gaaggccgtg tgcgtgctga agggcgacgg cccagtgcag 420
   ggcatcatca atttcgagca gaaggcaagg gctgggaccg ggaggcttgt gttgcgaggc 480
   cgctcccgac ccgctcgtcc ccccgcgacc ctttgcatgg acgggtcgcc cgccagggct 540
   agagcagtta agcagcttgc tggaggttca ctggctagaa agtggtcagc ctgggattgc 600
   atggacggat ttttccactc ccaagtctgg ctgcttttta cttcactgtg aggggtaaag 660
   gtaaatcagc tgttttcttt gttcagaaac tctctccaac tttgcacttt tcttaaagga 720
   aagtaatgga ccagtgaagg tgtggggaag cattaaagga ctgactgaag gcctgcatgg 780
   attccatgtt catgagtttg gagataatac agcaggtggg tcataattta gctttttttt 840
   cttcttctta taaataggct gtaccagtgc aggtcctcac tttaatcctc tatccagaaa 900
   acacggtggg ccaaaggatg aagagaggta acaagatgct taactcttgt aatcaatggc 960
   gatacgtttc tggagttcat atggtatact acttgtaaat atgtgcctaa gataattccg 1020
   tgtttccccc acctttgctt ttgaacttgc tgactcatgt gaaaccctgc tcccaaatgc 1080
   tggaatgctt ttacttcctg ggcttaaagg aattgacaaa tgggcactta aaacgatttg 1140
   gttttgtagc atttgattga atatagaact aatacaagtg ccaaagggga actaatacag 1200
   gaaatgttca tgaacagtac tgtcaaccac tagcaaaatc aatcatcatt tgatgctttt 1260
   catataggca tgttggagac ttgggcaatg tgactgctga caaagatggt gtggccgatg 1320
   tgtctattga agattctgtg atctcactct caggagacca ttgcatcatt ggccgcacac 1380
   tggtggtaag ttttcataaa ggatatgcat aaaacttctt ctaacagtac agtcatgtat 1440
   ctttcacttt gattgttagt cgcgaattct aagatccaga taaactgtgt ttctgctttt 1500
   aaactactaa atattagtat atctctctac taggattaat gttatttttc taatattatg 1560
   aggttcttaa acatcttttg ggtattgttg ggaggaggta gtgattactt gacagcccaa 1620
   agttatcttc ttaaaatttt ttacaggtcc atgaaaaagc agatgacttg ggcaaaggtg 1680
   gaaatgaaga aagtacaaag acaggaaacg ctggaagtcg tttggcttgt ggtgtaattg 1740
   ggatcgccca ataaacattc ccttggatgt agtctgaggc cccttaactc atctgttatc 1800
   ctgctagctg tagaaatgta tcctgataaa cattaaacac tgtaatctta aaagtgtaat 1860
   tgtgtgactt tttcagagtt gctttaaagt acctgtagtg agaaactgat ttatgatcac 1920
   ttggaagatt tgtatagttt tataaaactc agttaaaatg tctgtttcaa tgacctgtat 1980
   tttgccagac ttaaatcaca gatgggtatt aaacttgtca gaatttcttt gtcattcaag 2040
   cctgtgaata aaaaccctgt atggcactta ttatgaggct attaaaagaa tccaaattca 2100
   aactaaatta gctctgatac ttatttatat aaacagcttc agtggaacag atttagtaat 2160
   actaacagtg atagcatttt attttgaaag tgttttgaga ccatcaaaat gcatacttta 2220
   aaacagcagg tcttttagct aaaactaaca caactctgct tagacaaata ggctgtcctt 2280
   tgaagctt                                                          2288